# EUROPÄISCHE PATENTSCHRIFT

(11) **EP 2 817 002 B1**
(45) Veröffentlichungstag und Bekanntmachung des Hinweises auf die Patenterteilung: **27.06.2018**
(21) Anmeldenummer: 13704360.0
(22) Anmeldetag: 12.02.2013
(51) Int. Cl.: A61K 31/00, A61K 31/05, A61P 11/04, A61K 31/192, A61K 31/216, A61K 36/185, A61K 36/68, A61K 36/53, A61K 36/69, A61K 36/899, A61K 9/00, A61K 9/08, A61K 9/12, A61K 9/28

(54) **KOMBINATION AUS LIPIDTRANSFERPROTEINEN UND PHENOLDERIVATE ENTHALTENDEN PFLANZENSTOFFEN MIT LOKAL-MUCOSALER EFFIZIENZ**
COMBINATION OF LIPID TRANSFER PROTEINS AND PLANT MATTER CONTAINING PHENOL DERIVATIVES WITH LOCAL MUCOSAL EFFICIENCY
COMBINAISON DE PROTÉINES DE TRANSFERT LIPIDIQUE ET DE DÉRIVÉS PHÉNOLIQUES CONTENANT DES SUBSTANCES VÉGÉTALES LOCALEMENT EFFICACES SUR LES MUQUEUSES

(30) Priorität: 20.02.2012 DE 102012003286
(43) Veröffentlichungstag der Anmeldung: 31.12.2014
(73) Patentinhaber: Beutler, Rolf, 64739 Höchst im Odenwald (DE); Schmidt, Karlheinz, 72810 Gomaringen (DE)
(72) Erfinder: BEUTLER, Rolf, 64739 Höchst/Hummetroth (DE); SCHMIDT, Karlheinz, 72810 Gomaringen (DE)
(74) Vertreter: Müller, Claudia
(86) Internationale Anmeldenummer: PCT/EP2013/000410
(87) Internationale Veröffentlichungsnummer: WO 2013/124041

(56) Entgegenhaltungen:
- EP-A1- 0 303 227
- EP-A1- 1 829 548
- WO-A2-2007/059836
- NATALE SANTAGATI ET AL: "Simultaneous determination of catechins, rutin, and gallic acid in Cistus species extracts by HPLC with diode array detection.", JOURNAL OF CHROMATOGRAPHIC SCIENCE, Bd. 46, Nr. 2, 1. Februar 2008 (2008-02-01), Seiten 150-156, XP055058859, ISSN: 0021-9665
- CHAND N ET AL: "Mucolytic activity of azelastine in mice and rats", AGENTS AND ACTIONS, Bd. 38, Nr. 3-4, 1. März 1993 (1993-03-01) , Seiten 165-170, XP009168549, BIRKHAEUSER VERLAG, BASEL, CH ISSN: 0065-4299
- BASF: "Lutrol L and Lutrol F-Grades", Technical information , April 2010 (2010-04), XP002695043, Gefunden im Internet: URL:http://www.pharma-ingredients.basf.com /Statements/Technical%20Informations/EN/Ph arma%20Solutions/03_100102e_Lutrol%20L%20a nd%20Lutrol%20F-Grades.pdf [gefunden am 2013-04-09]
- Simonetta Caira, Rosa Pizzano, Gianluca Picariello, Gabriella Pinto, Marina Cuollo, Lina Chianese and Francesco Addeo: "Allergenicity of Milk Proteins", Milk Protein , September 2012 (2012-09), Seiten 173-214, XP002695044, DOI: 10.5772/52086 ISBN: 978-953-51-0743-9 Gefunden im Internet: URL:http://www.intechopen.com/books/milk-p rotein/allergenicity-of-milk-proteins [gefunden am 2013-04-09]

## Beschreibung

### Gegenstand der Erfindung

Die vorliegende Erfindung betrifft Kombinationen, insbesondere zur lokal-mucosalen Applikation, vor allem in fester, halbfester oder flüssiger Zubereitung, enthaltend LipidTransfer Proteine aus Pflanzen, Milch oder Kombinationen hiervon, mit jeweils einer mittleren Molekülmasse von 1-13 kDa, vor allem 1-9 kDa, vorzugsweise von 1-5 kDa, oder Milch mit einer mittleren Molekülmasse von 1-8 kDa, vor allem 1,5-7 kDa, sowie ein oder mehrere Phenolderivate enthaltende Pflanzenstoffe (Pulver oder Extrakte der Drogen), der nachfolgenden Formel, ausgewählt aus Pelargonium sidoides und/oder reniforme oder Mischungen hiervon, oder aus Kombinationen Thymian + Spitzwegerich; Thymian + Spitzwegerich + Anis; Thymian + Primelwurzel; Pelargonium (sidoides, reniforme oder Mischungen) + Thymian und/oder Zistrose; Spitzwegerich + Efeublätter; Thymian + Fenchel; Spitzwegerich + Löwenzahn, Pelargonium (sidoides, reniforme oder Mischungen) + Zistrose und/oder Senegawurzel, Pelargonium + Granatapfel. Derartige Kombinationen, insbesondere Zubereitungen hiermit, weisen vor allem eine lokal-mucosale Wirkung, insbesondere auf die Mund-, Hals- und Rachenschleimhaut z.B. bei Reizung, Trockenheit, Halitose, Infektionen, Allergien, Umweltnoxen oder sonstige Reizstoffe auf. Es hat sich gezeigt, dass durch Wechselwirkung der genannten Proteine mit den Phenolderivate enthaltenden Pflanzenstoffen Assoziate (Mucobione) entstehen. Überraschenderweise modifizieren diese Assoziate (Mucobione) gezielt die physikalischen Eigenschaften wässriger Systeme, zu denen auch physiologische Schleimschichten gehören, wie z.B. die Oberflächenspannung und damit auch die visco-elastischen Eigenschaften des Schleims. Dadurch wird erfindungsgemäß die Schleimmotilität verbessert. Hierdurch erfolgt ein erleichterter Abtransport korpuskulärer Materialien verschiedenster Art wie vor allem Stäube, allergene Partikel, Bakterien, Sporen etc. von den Schleimhäuten. Ferner bleibt die Wirkung lokal-mucosal begrenzt. Die erfindungsgemäßen Kombinationen und Zubereitungen eignen sich daher zur physikalischen Anwendung bei den genannten Indikationen auf Schleimhäuten.

### Stand der Technik

Zubereitungen mit Lipidtransferproteinen sind im Stand der Technik bekannt. So beschreibt die EP 1635776 A1 topisch applizierbare Zubereitungen, enthaltend Wasser, ein oder mehrere Lipide, ein oder mehrere Lipidtransferproteine aus Pflanzen (MW 4 bis 10 kDa) oder Milch (MW 2-4 KDa), sowie Hydrokolloide wie Gelatine, Acrylate u.ä. Diese Zubereitungen können insbesondere in Form einer Creme, eines Gels, Sprays, auf der Haut zum Lipidtransfer und damit der Verbesserung der Lipidstruktur von Haut eingesetzt werden. Je nach weiterer Indikation können auch Wirkstoffe wie Pflanzenextrakte hinzugesetzt werden. In der WO 2007/059836 A2 werden Systeme zum Recycling oxidativ veränderter Lipide aus diesen enthaltenden Strukturen wie Biomembranen (Haut) beschrieben, welche ein Depot aus einem oder mehreren strukturell/vor allem oxidativ nicht veränderten Lipiden, ein oder mehrere Recycling-Transferproteine mit Transferraten für strukturell/insbesondere oxidativ veränderte Lipide, einen oder mehrere nicht transferierbare Wirkstoffe zur Regenerierung strukturell/oxidativ veränderter Lipide; und ggf. einen oder mehrere anwendungsbezogene Zusatzstoffe aufweisen. Mit derartigen Zubereitungen kann das strukturell und vor allem oxidativ veränderte Lipid mittels des dafür vorgesehenen Transferproteins aus den diese enthaltenden Aggregaten herausgelöst, in das Depot mit nicht veränderten Lipiden transferiert, und durch die hier vorhandenen Wirkstoffe regeneriert sowie anschließend rücktransferiert werden. Als Recycling- Transferproteine werden vor allem Buchweizen, Hirse, Dinkel, Tapioka mit einer mittleren Molekülmasse von 3,5 bis 8 kDa, bzw. Milch mit einer mittleren Molekülmasse von 2,5 bis 8 kDa und einer Transferrate für oxidierte Lipide von 600 bis 8000 ng/ min/mg Protein, vorgeschlagen.

Lipidtransferproteine sind aus Pflanzen oder tierischen Quellen erhältliche Proteine mit einer z. B. gegenüber Kollagen/Keratin geringeren Molekülmasse. Sie weisen eine Transferaktivität gegenüber speziellen Lipiden wie Phosphatidylcholin auf (spezifische Lipidtransferproteine, z. B. aus Maissamen, Spinatblättern (siehe D. Zilversmit, Journal of Lipid Research, Vol.25, 1984, 1563 - 1569). Daneben existieren auch nicht spezifische Lipidtransferproteine mit breiterem Transferspektrum für mehrere Lipide wie z. B. Proteine aus Rizinusbohnen, Weizen, Reis, Spinat, Gerste, Raps, Karotten, siehe J.C. Kader, Trends in plant Science, Februar 1997, Vol. 2, S. 1330-35.

Zoccatelli, G. et al, J. Agric. Food Chem. 2007, 55, Seite 11057-11062 beschreiben die Isolierung und Identifizierung von 2 speziellen niedermolekularen (7-9 kDa) Lipidtransferproteinen aus Granatapfel, welche in das allergische Potential dieser Frucht involviert sein sollen.

EP 0 303 227 A1 betrifft die Herstellung neuer Phenyloxy- bzw. -Phenyl-thioether-Verbindungen, welche eine entzündungshemmende und eine die Bronchialsekretion verflüssigende Wirkung angenommen wird.

Chand et al, Agents and Actions, Birkhaeuser Verlag, Basel, CH, Bd. 38, Nr. 3-4, 1 März 1993, Seiten 165-170 beschreiben in dem Artikel "Mucolytic activity of azelastine in mice and rats" den Nachweis der entzündungshemmenden und auch den Bronchialschleim verdünnenden Wirkung von Azelastin als Antihistaminwirkstoff.

Pflanzenstoffe z.B. aus Blättern, Zweigen, Samen oder auch Extrakte daraus sind vielfach bekannt und als Arzneimittel erprobt. Sie umfassen insbesondere ätherische Öle aus der Gruppe der Terpene, Terpenalkohole/-aldehyde sowie der Phenolderivate wie -alkohole, -säuren, -ester, -aldehyde. So eignen sich z. B. Thymian, Spitzwegerich oder Pelargonium (sidoides, reniforme), vor allem Kombinationen aus letztgenannten, zur Behandlung von Rachenerkrankungen, wie in der EP 1829548 B1 beschrieben. Hierin werden insbesondere Lutschpastillen beschrieben. Dabei wird bzgl. Pelargonium sidoides die Wirkung der darin enthaltenden Gerbstoffe (Catechine/Gallussäure=Phenol-Derivat) durch physikalische Vernetzung mit Schleimhauteiweißen angenommen. Es handelt sich hier also um eine der Gerbung analoge Wechselwirkung der pflanzlichen Phenolderivate mit den mucosalen Polymerproteinen (Mucinen). Spitzwegerich (umfassend Terpenalkohol, Phenolester, Kohlenhydrate) soll aufgrund der Bindung der darin enthaltenden Polysaccharide, insbesondere der Glucomannane (Kohlenhydrate), an die Schleimhaut, quasi als Ersatz für infektions- oder entzündungsbedingt fehlende native Mucopolysaccharide, eine Wirksamkeit entfalten.

Das beschriebene Prinzip des Zusammenwirkens einer Schleimstoffdroge und einer Gerbstoffdroge wird ebenfalls in der WO 2010/00 3472 A2 aufgegriffen. Hierbei werden spezielle Pastillen aus einer äußeren festen Hülle, enthaltend eine Gerbstoffdroge (wie Pelargonium) und einem inneren flüssigen Kern, enthaltend die Schleimdroge (wie Spitzwegerich), vorgeschlagen. Mit dieser Aufteilung der Wirkstoffe in 2 Phasen soll eine zeitversetzte Freisetzung und damit verbesserte Wirkung erfolgen. Mit den oben beschriebenen Wirkstoffkombinationen können somit lokal/topisch entzündliche Prozesse im Schleimhautbereich behandelt werden.

Eine einphasige Kombination dieser Wirkstoffgruppen scheint weniger günstig, auch wegen der bekannten Unverträglichkeiten der Inhaltsstoffe.

Darüberhinaus ist eine kontrollierte Wechselwirkung der Phenolderivate aufgrund der beschriebenen Vernetzungsreaktionen nicht möglich, da nicht klar ist, mit welchen Mucoproteinen eine Reaktion erfolgt. Dies kann zu weiteren, ggf. auch nicht erwünschten pharmakologischen (z.B. systemischen), immunologischen und metabolischen Wirkungen führen.

### Aufgabe vorliegender Erfindung

Trotz des oben beschriebenen Fortschritts der überraschenden Kombinierbarkeit von Gerbstoff- und Schleimstoffdrogen für den beschriebenen Anwendungsbereich wäre es von Vorteil, wenn Zubereitungen vorlägen, worin die Effektivität der Wirkstoffe nicht zwingend von deren zeitlich/lokaler Anordnung bzw. Freisetzung abhinge. Auch wäre es wünschenswert, wenn ein kontrollierbarer Effekt der pflanzlichen Wirkstoffe herbeigeführt werden könnte, um unerwünschte Folgereaktion wie beschrieben gezielt zu vermeiden.

### Lösung der Aufgabe

Diese Aufgabe wird erfindungsgemäß gelöst durch lokal, insbesondere mucosal vor allem oral einsetzbare Kombinationen aus einem oder mehreren Phenol-Derivate-haltigen Pflanzenstoffen (wie Pulver, Extrakte aus Pflanzen) gemäß nachfolgender Formel, ausgewählt aus Pelargonium sidoides und/oder reniforme oder Mischungen hiervon, oder aus Kombinationen Thymian + Spitzwegerich; Thymian + Spitzwegerich + Anis; Thymian + Primelwurzel; Pelargonium (sidoides, reniforme oder Mischungen) + Thymian und/oder Zistrose; Spitzwegerich + Efeublätter; Thymian + Fenchel; Spitzwegerich + Löwenzahn, Pelargonium (sidoides, reniforme oder Mischungen) + Zistrose und/oder Senegawurzel, Pelargonium + Granatapfel, zusammen mit einem oder mehreren Lipidtransferproteinen, ausgewählt aus einem oder mehreren Pflanzenproteinen, mit jeweils einer mittleren Molekülmasse von 1 bis 13 kDa, vor allem 1 bis 9 kDa, vorzugsweise von 1 bis 5 kDa, oder Milchprotein mit jeweils einer mittleren Molekülmasse von 1 bis 8, vorzugsweise 1,5 bis 7, vor allem 1,5 bis 5 kDa, oder Kombinationen hiervon aus Pflanzen- und Milchprotein(en), welche einen pH-Wert von 4 bis 6,5 (bei 20° C, 10%ige wässrige Lösung) aufweisen.

Es ist bevorzugt, wenn das oder die Lipidtransferproteine ausgewählt ist/sind aus Hafer, Hirse, Weizen, Gersteprotein, jeweils mit einer mittleren Molekülmasse von 1 bis 9 kDa, oder Kombinationen hiervon, oder aus einem oder mehreren Milchproteinen mit einer mittleren Molekülmasse von 1,5 bis 7 kDa, vor allem 1,5 bis 4,5 kDa, oder Kombinationen hiervon mit Pflanzenproteinen.

Insbesondere geeignet sind Kombinationen aus den vorgenannten bevorzugten Phenolderivat- haltigen Pflanzenstoffen und den vorgenannten bevorzugten Transferproteinen, sowie Zubereitungen, enthaltend diese. Dazu gehören Pelargonium sidoides und/oder reniforme bzw. Extrakte hieraus wie nachfolgend beschrieben, insbesondere in Kombination mit Haferprotein, Weizenprotein, Gersteprotein, Milchprotein oder Kombinationen aus Hafer- und Milchprotein, Weizenprotein- und Milchprotein, Gersteprotein und Milchprotein, Hafer-, Gerste oder Weizenprotein und Milchprotein der jeweils oben beschriebenen Molekülmassen. Das Verhältnis der Proteine zueinander kann variieren, bevorzugt ist das Verhältnis von Pflanzenprotein(en, gesamt) zu Milchprotein(en, gesamt) 1:1 bis 1:3.

Die Mengenangaben beziehen sich auf Gewichtsprozent, soweit nicht anders angegeben. Die Verhältnisangaben beziehen sich auf Gewichtsprozent der vorhandenen Substanzmengen (ohne ggf. in den Extrakten, Pulvern vorhandene Zusätze).

Der oder die Pflanzenextrakt(e) können in Form eines Fluids, eines Spissums, eines Trockenextraktes/festen Pulvers und das oder die Transferproteine bevorzugt als festes Pulver vorliegen.

Die Pflanzen(Drogen)extrakte sind bevorzugt Flüssigextrakte, z. B. auch in Form einer Urtinktur, vor allem mit einem Droge/Extrakt- Verhältnis von 1:0,1 bis 1:100, vor allem 1:0,5 bis 1: 40, insbesondere 1:3 bis 1:13.

Auch Trockenextrakte sind erfindungsgemäß besonders geeignet, z. B. solche mit einem Droge/Extrakt-Verhältnis (DEV) von 100:1 bis 1:1, vor allem 60:1 bis 30:1, insbesondere 30:1 bis 10:1.

Die anmeldungsgemäßen Kombinationen können vor allem in Zubereitungen eingesetzt werden, welche weiterhin Trägerstoffe, vor allem in Mengen von 60 bis 95 Gew.%, sowie ggf. Zusatzstoffe wie nachfolgend beschrieben, aufweisen.

Überraschenderweise hat sich gezeigt, dass die durch Assoziatbildung aus den verwendeten Transferproteinen und den phenolischen Derivaten der Pflanzenstoffe gebildeten Mucobione zu einer gezielten Modifikation der physikalischen Eigenschaften der Schleimschicht führen. Insbesondere erfolgt eine Absenkung der Oberflächenspannung. Dies bedingt Veränderungen der visco-elastischen Eigenschaften des Schleims und damit eine verbesserte Schleimmotilität mit der Folge des erleichterten Abtransports korpuskulärer Materialien wie oben beschrieben. Da die Phenolderivate spezifisch mit den anmeldungsgemäßen Transferproteinen assoziiert sind, erfolgt - im Gegensatz zu bisherigen Reaktionsfolgen- eine kontrollierte lokal physikalische Wirkung. Dabei ist es nicht erforderlich, scheinbar unverträgliche Wirkstoffe räumlich voneinander zu trennen, da die eingesetzten Proteine spezifisch mit den Phenolderivaten in Wechselwirkung treten. Dies ist insbesondere auch deshalb überraschend, weil der Stand der Technik den Transfereffekt vor allem auch mit Lipiden oder amphiphilen Stoffen beschreibt. Darüber hinaus konnte nicht erwartet werden, dass die anmeldungsgemäßen Proteine überhaupt einen Einfluss zeigen, da bekanntlich die Mucine der Schleimhaut (dabei handelt es sich um anionische Glyko-Proteine mit einer zentralen Protein-Kette und langen Polysaccharidketten) selbst Addukte mit den phenolischen Wirkstoffen bilden können (s.o.). Ohne an eine Theorie gebunden zu sein, wird angenommen, dass es durch die gezielt eingesetzten Transferproteine zu einer Absättigung der phenolischen Derivate der Pflanzenstoffe kommt und dadurch unspezifische Wechselwirkungen mit den Schleimhautmucinen und damit unerwünschte pharmakologische, immunologische oder metabolische Effekte vermieden werden können.

Die anmeldungsgemäßen Kombinationen bzw. Zubereitungen sind daher geeignet zum Einsatz bei Reizungen, Rötungen, Entzündungen, z.B. durch Allergene, Lufttrockenheit, Staubpartikel, Bakterien, Sporen der Mund-, Hals- und Rachenschleimhaut, aber auch der Nasenschleimhaut oder der Bronchialschleimhaut.

Bevorzugte Zubereitungsformen sind flüssige Zubereitungen (Kombinationen wie beschrieben mit Wasser als Hauptträgerstoff, d.h. mehr als 60 Gew.%) wie Tropfen, Saft, Lösungen (zum Sprühen, Gurgeln, Spülen), lutschbare Pastillen (mit Zucker/Zuckeraustauschstoffen als Hauptträgerstoff) oder kaubare feste Darreichungsformen wie Kautablette, Medicated Gum (mit Dextrose oder Chicle /Wasser als Träger).

### Abbildungen

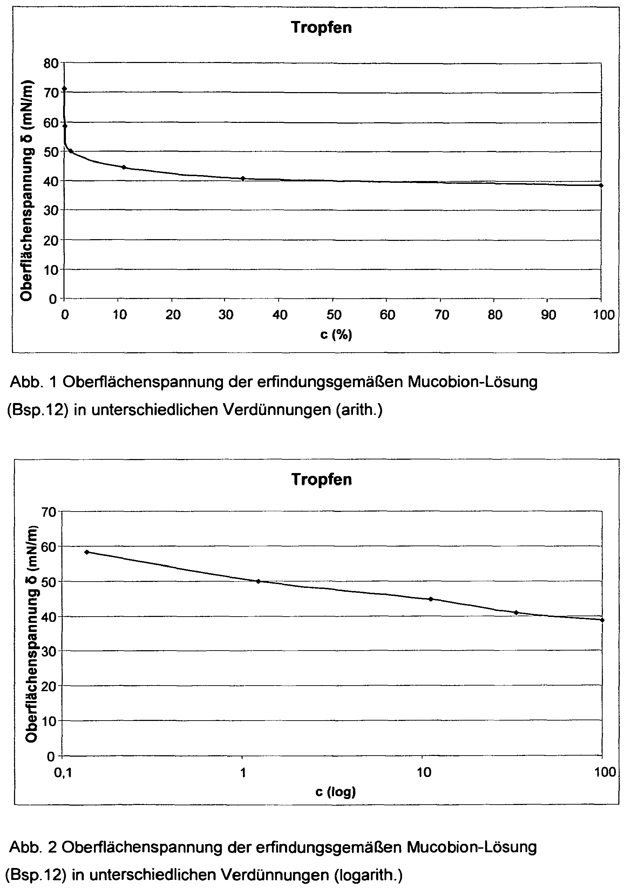

Abb.1 und 2 zeigen die graphische Darstellung der Meßergebnisse gemäß Beispiel 8 und 9 und, woraus sich die Erniedrigung der Oberflächenspannung wässriger Medien (wie z. B. Schleimhäute) mit den anmeldungsgemäßen Mucobion-Assoziaten selbst bei hoher Verdünnung ergibt.

### Nähere Erläuterung der Erfindung

Die Erfindung betrifft Zubereitungen, insbesondere in fester, halbfester oder flüssiger, vor allem oral/lokal einsetzbarer Form, umfassend eine Kombination aus Phenol-Derivate-haltige Pflanzenwirkstoffen wie beschrieben (z.B. 0,01 bis 10 Gew.%) zusammen mit Transferproteinen (z.B. 0,01 bis 10 Gew.%), durch deren Assoziatbildung Mucobione entstehen. Vorzugsweise sind 0,1 bis 15 Gew.%, vor allem 0,5 bis 10 Gew.%, eines oder mehrerer Phenol- und oder Phenol- Derivate-haltiger Pflanzenstoffe (Pulver, Extrakte) oder Kombinationen hiervon wie beschrieben vorhanden, vor allem ausgewählt aus der Gruppe, umfassend Pflanzenwirkstoffe mit folgender Spezifikation:
Pelargonium (sidoides und/oder reniforme) mit Gallussäure/-ester, Polyphenolen;
Spitzwegerich (Plantago lanceolata) mit Oct-1-en-3-ol, ortho-Dihydroxyzimtsäure;
Granatapfel (Punica granatum) mit Gallussäure;

Thymian (Thymus vulgaris) mit Thymol, Carvacrol, Ursolsäure (Terpeoide) sowie Rosmarinsäure, Kaffeesäure (Phenolsäuren, -ester), nicht jedoch Catechinen oder hydrolysierbaren Labiaten.

Zistrose (Cistus ladanifer, Cistus creticus, Cistus incanus ssp. tauricus) mit Polyphenolen, insbesondere 3,4 Dihydroxybenzoesäure, Procyanidinen, Flavan-3-olen; Labdanum-Öl; Senega (Klapperschlangenwurz) mit Phenolglykosiden (4-(Di)Methoxyzimt-säure-Fucose-Derivat (Tetrasacharid aus Fucose, Glucose, Rhamnose, Xylose, Galactose, mit Triterpenoid-Rest derivatisiert: Presenegenin.

Weiterhin umfasst die Kombination in den erfindungsgemäßen Zubereitungen:
0,1 bis 15 Gew.%, insbesondere 0,3 bis 10 Gew.% eines oder mehrerer Lipidtransferproteine, ausgewählt aus einem oder mehreren Pflanzenproteinen, ausgewählt aus Hafer, Gerste, Weizen, oder Milchproteinen, oder Kombinationen hiervon, mit jeweils einer mittleren Molekülmasse von 1 bis 13 kDa, vor allem 1 bis 9 kDa, vorzugsweise von 1 bis 5 kDa, oder Milchprotein mit jeweils einer mittleren Molekülmasse von 1 bis 8 kDa, vorzugsweise von 1,5 bis 7 kDa, insbesondere 1,5 bis 5 kDa, oder auch 1,8 bis 3,5 kDa, welche einen pH-Wert von 4 bis 6,5 bei 20° C, 10%ige wässrige Lösung aufweisen. In den Zubereitungen liegt vorzugsweise ein Verhältnis von Phenolderivate enthaltendem Pflanzenstoff zu Protein von 0,1:1 bis 1:10, insbesondere 0,5:1 bis 1:10, vor allem 1:1 bis 1:7 vor. Das Verhältnis bezieht sich auf die Substanzmengen (ohne eventuell vorhandene Zusätze).

Die Zubereitungen umfassen weiterhin einen Träger, ausgewählt aus Wasser, einem oder mehreren Zuckern, Zuckeraustauschstoffen oder Mischungen aus Zucker/Zuckeraustauschstoffen und Wasser. Die Zucker/ Zuckeraustauschstoffe können gewählt werden aus Glucose, Maltose, Dextrose, Isomaltose, Fructose, Xylose, Lactose, Lactulose, Galactose, Inulin, Maltitol, Sorbitol, Xylitol, Mannitol, Erythritol, oder Mischungen hiervon, d.h. Mischungen aus verschiedenen Zuckern oder auch aus einem oder mehreren Zuckern und Wasser, insbesondere Sirupe mit einer Zuckerkonzentration von z.B. 50 bis 70 Gew. % wie Glucose- oder Saccharosesirup.

Die Trägerstoffe (z. B. 60 und mehr Gew.%) sind vor allem in Mengen von 72 bis 98 Gew. % vorhanden.

Die anmeldungsgemäßen Kombinationen können weiterhin Zusatzstoffe, insbesondere pharmazeutische zulässige Zusatzstoffe aufweisen, ausgewählt aus Lösungsmitteln, Geschmacks- und Aromastoffen, Säuerungsmitteln, Puffersubstanzen, Süßstoffen, Stabilisatoren, Zusatzwirkstoffen oder Kombinationen hiervon.

Mit den anmeldungsgemäßen Kombinationen (Mucobionen) werden vor allem in Verbindung mit Trägerstoffen und ggf. Zusatzstoffen Zubereitungen erhalten, die zur Anwendung bei Schleimhautirritationen wie Entzündungen, allergischen Reaktionen, Reaktionen auf Umweltpartikel, Staub, Infekte, Reizungen/Entzündungen durch bakterielle und/oder virale Organismen eingesetzt werden können. Hierzu gehören Reizungen/Entzündungen im Hals-Rachenraum, im Nasen- und Bronchialtrakt, auch im intestinalen oder vaginalen Bereich. Dazu können sie in Abhängigkeit der jeweils geeigneten bzw. zugelassenen weiteren Bestandteile (Zusatzstoffe) für Darreichungsformen insbesondere als Medizinprodukt, aber auch als Arzneimittel vorliegen. Insbesondere umfassen erfindungsgemäße Zubereitungen biologische Inhaltsstoffe. Hierunter werden gemäß vorliegender Anmeldung solche Substanzen verstanden, die in der Natur vorkommen/wachsen und welche als solche in/ aus dieser abbaubar sind.

### Nähere Erläuterung der Inhaltsstoffe der anmeldungsgemäßen Zubereitungen

### 1. Phenolische (Phenolderivate enthaltende) Pflanzenwirkstoffe

Die erfindungsgemäßen Zubereitungen enthalten als ersten wirksamen Bestandteil ein oder mehrere Pflanzenstoffe mit Phenolischen Inhaltsstoffen (Phenolderivate) der allgemeinen Formel: 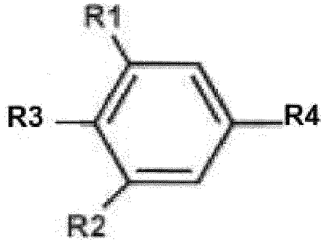 worin R₁, R₂, R₃, R₄, R₅ unabhängig voneinander bedeuten:
R₁: H, OH, OR₅, COR₅; C₁-C₈Alkyl, C₁-C₈substituiertes Alkyl;
R₂: OH; OR₅, H; C₁-C₈Alkyl, C₁-C₈substituiertes Alkyl;
R₃: OH; OR₅, C₁-C₈Alkyl, C₁-C₈substituiertes Alkyl; oder Zucker gemäß R_{4,5};
R₄: COOR₅; C₁-C₈ -Alkyl, C₁-C₈-Alkenyl-COOR₅; C₁-C₈Alkyl, C₁-C₈substituiertes Alkyl;
R₅: H, C₁-C₈-Alkyl, COOC₁-C₈ alkyl-subst.Phenol, Zucker, ausgewählt aus Glucose, Fructose, Fucose, Xylose, Galactose, Rhamnose, Xylose und Kombinationen aus 2 oder mehreren dieser Zucker; wobei die Zucker mit Mono-Di-Triterpenen assoziiert sein können,
wobei der Pflanzenwirkstoff ausgewählt ist aus Pelargonium sidoides und/oder reniforme oder Mischungen hiervon oder Kombinationen aus Thymian + Spitzwegerich; Thymian + Spitzwegerich + Anis; Thymian + Primelwurzel; Pelargonium (sidoides, reniforme oder Mischungen) + Thymian und/oder Zistrose; Spitzwegerich + Efeublätter; Thymian + Fenchel; Spitzwegerich + Löwenzahn, Pelargonium (sidoides, reniforme oder Mischungen) + Zistrose und/oder Senegawurzel, Pelargonium + Granatapfel.

Zu den erfindungsgemäß eingesetzten Wirkstoffen gehören jene, worin die Phenolderivate folgende Gruppen aufweisen: R₁, R₂ = OH und R₄=COOH (Gallussäure, wie in Pelargonium, Granatapfel); R₁ =H, R₃ =OH und R₄ = C₂-Alkenyl-COOR₅ = mit R₅ = H oder C₁-C₈-Alkyl (Kaffeesäure/ester) bzw. R₁ =H, R₂ =OH, R₃ = subst. C₁-C₈-Alkyl; R₄ = C₁-C₈-Alkyl wie Thymol / Thymian.

Weiterhin können Wirkstoffe eingesetzt sein, worin R₄ COOH und R_{1,2} OH, OCH₃ ist wie Zistrose mit Dihydroxybenzoesäure; bzw. Senegawurzel mit R₄: C₁-C₄-Alkenyl-COOR₅ mit R₅= =Triterpenoid-Tetraglycosid,s.o.) und R_{2,3}=OCH₃ bzw. Spitzwegerich R₄: C₁-C₄-Alkenyl-COOR₅, mit R₂, R_{3,}=OH, R₅= OCH₃ (mit Zimtsäure).

Vorzugsweise werden Phenolderivate-haltige Pflanzenstoffe ausgewählt aus Pelargonium sidoides und/oder reniforme oder Mischungen hiervon; insbesondere ergänzt durch einen oder mehrere Wirkungsverstärkende (vor allem Terpenoide Substanzen aufweisende) Mittel (Zusatzwirkstoffe), ausgewählt aus der Gruppe, umfassend Löwenzahn, Primelwurzel, Fenchel, Anis, Efeu, Isländisch Moos, Cranberry, oder geeignete Mischungen hiervon aus 2, 3 oder 4 verschiedenen Mitteln dieser Gruppe.

Insbesondere geeignet sind Kombinationen aus Thymian + Spitzwegerich; Thymian + Spitzwegerich + Anis; Thymian + Primelwurzel; Pelargonium (sidoides, reniforme) + Thymian und/oder Cistus; Spitzwegerich + Efeublätter; Thymian + Fenchel; Spitzwegerich + Löwenzahn/oder Cranberry.

Besonders bevorzugte Kombinationen und Zubereitungen weisen auf:
Pelargonium sidoides, reniforme (oder Mischungen)- Fluid- Extraktpulver (z.B. 10-70% nativ, Extraktionsmittel: Wasser-Ethanol, 10 bis 15%, Zusatz im Pulver: Maltodextrin) zusammen mit Haferprotein, MW 1,5 bis 2 kDa, insbesondere. 1,8 kDa (z.B. 70 bis 90%, Pulver), Haferprotein (Pulver MW 1 bis 5 kDa, vor allem. 1 kDa, Pulver, z.B. 20 bis 70%) oder eine Kombination aus Haferprotein und Milchprotein dieser Art, vor allem in einem Gewichtsverhältnis der Proteine zueinander von 1:1.

Insbesondere beträgt hier das Verhältnis von Pelargonium-Pflanzenstoff zu Protein 0,1:1 bis 1:8, bevorzugt 0,4:1 bis 1:5, vor allem auch 1:1 bis 1:3. Das Verhältnis Protein/Pflanzenstoff bezieht sich auf das Reingewicht der Substanzgruppe ohne ggf. dabei vorhandene Substanzen (Verhältnis Proteinsubstanz zu Pflanzenstoffmenge).

Die Phenolderivate enthaltenden Pflanzenstoffe werden insbesondere in Form von Extrakten aus den Blättem/Wurzeln/Blüten der Pflanzen entweder als Pulver oder Fluidextrakte eingesetzt. Alternativ können auch Pulver aus den Blättern, Blüten, Wurzeln direkt nach entsprechender, dem Fachmann bekannter Aufarbeitung (Trocknen, Reinigen, Mahlen) eingesetzt werden.

Extrakte sind konzentrierte Zubereitungen von flüssiger, fester oder zähflüssiger Konsistenz, die normalerweise aus getrocknetem pflanzlichem oder tierischem Material hergestellt werden, wobei zuvor eine Vorbehandlung wie Mahlen, Entfetten, Inaktivierung von Enzymen (falls vorhanden) erfolgen kann.

Zähflüssige Extrakte, Dickextrakte sind Spissa, deren Konsistenz zwischen der von Fluid- und Trockenextrakten liegt. Sie werden durch teilweises Eindampfen des Lösungsmittels (wie Ethanol, Wasser oder Mischungen) erhalten (Trockenrückstand z.B. mindestens 70%,m/m).

Trockenextrakte (Sicca) sind feste Zubereitungen, die durch Verdampfen des zu ihrer Herstellung eingesetzten Lösungsmittels erhalten werden (Trockenrückstand z. B. mindestens 95 %,m/m).

Fluidextrakte (Fluida) sind flüssige Zubereitungen, von denen ein Masse- oder Volumenteil im allgemeinen einem Masseteil der getrockneten Ausgangsdroge entspricht. Diese Zubereitungen können bzgl. des Lösungsmittelgehalts/Gehalts an Inhaltsstoffen bzw. des Trockenrückstandes eingestellt werden (Droge-Extrakt-Verhältnis).

Tinkturen (Tincturae) sind flüssige Zubereitungen, die aus getrocknetem Ausgangsmaterial hergestellt werden, wobei eine Vorbehandlung wie genannt stattfinden kann. Eine Übersicht über die Herstellung von Extrakten und Drogenzubereitungen ist in Gaedcke, Steinhoff, "Phytopharmaka", Wissenschaftliche Verlagsgesellschaft mbH, Stuttgart, 2000, insbesondere Seite 4 gegeben.

Derartige Verfahren sind dem Fachmann bekannt. Es werden dementsprechend bekannte Pflanzenzubereitungen geeigneter Konsistenz und Konzentration im Handel angeboten. Sie entsprechen den Anforderungen an derartige Mittel im Bereich der MedizinproduktArzneistoffe.

Die anmeldungsgemäßen Phenolderivate enthaltenden Pflanzlichen Stoffe sind insofern insbesondere Pflanzen, deren Blätter, Zweige, Blütenschäfte oder Wurzeln in geeigneter (bekannter) Weise zum Einsatz in den beschriebenen Zubereitungen, vor allem zur Verwendung in Medizinprodukten oder auch pharmazeutischen Zubereitungen für medizinische Zwecke nach bekannten Verfahren aufgearbeitet werden und im Handel erhältlich sind.

Beispielhaft können folgende Gewinnungsmethoden genannt werden:
Die jeweils geeigneten Pflanzenteile werden durch Extraktion mit Lösungsmitteln wie Wasser, Alkohol (Ethanol, Glycerol oder geeignete Mischungen hiervon), ggf. unter Zusatz geeigneter Fällungsmittel (Ammoniaklösung)/organischer Lösungsmittel (wie Kohlenwasserstoffe, Triglyceride) entweder als Fluidextrakt oder als Liquidextrakt oder als Pulver erhalten. Die Methoden hierzu sind dem Fachmann bekannt.

Alternativ können die erforderlichen Phenol- Derivate, sofern sie isoliert zur Verfügung stehen, direkt als solche z. B. in Form von Pulvern eingesetzt werden. Die isolierten Phenolderivate können vorzugsweise in einer geringeren Menge als die Pflanzenstoffe selbst eingesetzt werden wie z. B. 0,001 bis 1 Gew.%, bezogen auf die Gesamtzubereitungen. Die Menge an Transferproteinen kann dann entsprechend angepasst werden, wie 0,01 bis 10 Gew.%, in Verhältnissen wie vorstehend beschreiben. In derartigen Zubereitungen werden dann Träger-und Zusatzstoffe sofern gewünscht inkorporiert.

Direktgewinnung von Pflanzlichen Inhaltsstoffen ist z. B. bekannt für Thymol, Zitronenöl, Pfefferminzöl (Menthol), Eukalyptusöl, die auch als Aromastoffe einsetzbar sind und durch Wasserdampfdestillation aus den Grundstoffen erhalten werden.

Pulverextrakte oder Wurzelextrakte wie solche aus Pelargonium sidoides können nach dem Trocknen/Mahlen der Blätter oder Zweige durch Extraktion mit Lösungsmitteln wie Wasser, Alkohol, organischen Lösungsmitteln oder Gemischen hiervon und nachfolgender Trocknung gewonnen werden.

Blütenextrakte können durch Extraktion mit Alkohol, vor allem Ethanol, oder Alkohol (insbesondere Ethanol)/Wasser- Gemischen und nachfolgende bekannte Aufarbeitung gewonnen werden.

Auch hier sind dem Fachmann die gängigen Herstellungsmethoden bekannt.

Die wie beschrieben gewonnenen Extrakte/Pulver werden je nach Wirksubstanz in bekannten Mengen verwendet.

Im Folgenden werden exemplarisch einige Extrakte/Herstellungsmethoden gemäß Stand der Technik beschrieben:
So kann Thymian- Fluidextrakt (z. B: DAB10) erhalten werden durch Behandeln des Krautes mit 10%iger wässriger Ammoniaklösung, 85%igem Glycerin, 90%igem Ethanol, Wasser (1:20:70:109). Nach Mazerisierung (5 Tage, RT), Filtration und Abpressen wird der Extrakt mit einem Gehalt an Phenolen und Thymol erhalten.

Ein Liquid-Extrakt kann analog gewonnen werden, jedoch unter Einsatz von 96%igem Ethanol, während der Thymian-Trockenextrakt einen 70%igen wässrigen Extrakt z. B. aus Herba Thymi vulgaris (3-6:1), 15% Maltodextrin, 15% Gummi arabicum darstellt. Pelargonium sidoides oder reniforme kann erhalten werden wie folgt:
Pelargonium Pflanzen Extrakte (z. B. aus Pelargnium sidoides) werden zum Einsatz in Medizin-Arzneiprodukten vorzugsweise gewonnen durch alkoholische Extraktion, z. B. mit Wasser/Ethanol. Dadurch kann z. B. ein Produkt gewonnen werden mit 10-15, vor allem 11 oder 15 % Ethanol (Droge-Extrakt Verhältnis von 1: 8-10).

Es können pro Tag 60 mg nativer Pelargonium sidoides Extrakt (Arzneimittel) oder 30 mg (Medizinprodukte = 50 % der Arzneimitteldosierung) eingesetzt werden.

Ganz besonders bevorzugt sind in anmeldungsgemäßen Kombinationen und Zubereitungen, vor allem auch im Zusammenhang mit den vorgenannten bevorzugten Proteinen, Pelargonium sidoides und/oder reniforme Wurzelextrakte mit einem Droge/Extrakt-Verhältnis von 3:1 bis 30:1, wie z.B. 7-25:1, vor allem mit Wasser/Ethanol (Wasser mit 10 bis 50% Ethanol, vor allem 15%).

Geeignet ist auch ein Trockenextrakt-Pulver hieraus, insbesondere mit 10 bis 70 Gew.% nativem Extrakt, bevorzugt 10 Gew.%.

Weiterhin bevorzugt sind Thymianextrakte (Herba Thymi vulgaris Ph.Eur.), mit einem Droge/Extrakt-Verhältnis von 3-6:1, mit Wasser als Auszugsmittel, insbesondere als Trockenpulver (wie 30 -80%, insbesondere 70% nativ).

Als Zusätze in den genannten Trockenextrakt-Pulvern sind vor allem Maltodextrin, Gummi arabicum Ph.Eur. geeignet.

Zur Herstellung eines geeigneten Spitzwegerich-Wirkstoffproduktes kann von zerkleinertem Kraut (Plantaginis lanceolatae herba) ausgegangen werden. Daraus können Mazerate, Fluidextrakte, Trockenextrakte, Sirupe (durch Lösen des Trockenextraktes) erhalten werden. Es kann auch je nach Applikationsart der Press-Saft der frischen Pflanze verwendet werden.

Ein bekanntes und besonders geeignetes Produkt ist hier ein nativer wässriger Spitzwegerich-Extrakt mit einem Droge-Extrakt- Verhältnis (DEV) von 4-6:1. Daraus lässt sich ein Trockenextrakt herstellen, bestehend aus 75% nativem Extrakt und 25% Maltodextrin. Die mittlere Tagesdosis für Erwachsene liegt zwischen 3-6 g (ethanolischer Fluidextrakt: 4,5 mg).

Senega (Polygala senega L.) wird durch Extraktion der zerkleinerten Wurzel einschließlich des Wurzelkopfes mittels Ethanol als ethanolischer Trockenextrakt (Polygalae extractum ethanolicum siccum oder Polygalae extractum ethanolicum siccum normatum PH) oder als Fluid (wie Polygalae extractum ethanolicum liquidum) bzw. als dickflüssigeres Produkt wie Polygalae sirupus PH.

Diese Produkte sind anmeldungsgemäß geeignet.

Zistrose (Cistus) kann als ethanolischer, ethanolisch-wässriger (z.B. 10-50:90-50) oder wässriger Primär-Extrakt (z.B. Droge/Extrakt-Verhältnis 1:3-6) oder als aus diesem Primärextrakt durch Lösungsmittelverdampfung als Sirup oder auch als Pulverextrakt, z. B. 80% nativ erhalten werden.

Isländisch Moos kann erhalten werden als Spissumextrakt aus z.B. dem ganzen oder geschnittenen Thallus von Cetraria islandica L. Acharius, mit einem Droge-Extrakt-Verhältnis z. B. von 2-5:1, wie vor allem 3:1 (Auszugsmittel: Wasser).

Granatapfel kann als eingedickter Saft oder Extrakt hieraus wie oben beschrieben gewonnen werden. Ggf. kann hier eine Sprühtrocknung erfolgen, so dass der das Phenolderivat enthaltende Pflanzenstoff als Pulver eingesetzt werden kann.

Die beschriebenen Pflanzenextrakte/Pulver weisen die genannten Phenolderivate in Mengen von 0,05 bis 5 Gew.% bzw. einen Gesamtpolyphenolgehalt von 0,5 bis 25 Gew.% auf. Üblicherweise sind daneben auch noch weitere Inhaltsstoffe enthalten.

### 2) Transferproteine

Die erfindungsgemäßen Zubereitungen enthalten als zweiten wirksamen Bestandteil ein oder mehrere Lipidtransferproteine, ausgewählt aus einem oder mehreren Pflanzenproteinen, ausgewählt aus Hafer, Hirse, Weizen, oder auch Gerste, Algen, mit jeweils einer mittleren Molekülmasse von 1 bis 13 kDa, vor allem 1 bis 9 kDa, vorzugsweise von 1 bis 5 kDa, oder Milchproteinen mit einer mittleren Molekülmasse von 1 bis 8, vorzugsweise 1,5 bis 7, insbesondere 1,5 bis 5 kDa, oder Kombinationen hiervon, welche einen pH-Wert von 5 bis 6,5 bei 20° C, 10%ige wässrige Lösung aufweisen. Die Pflanzenproteine werden gewonnen durch Aufschlämmung, Zentrifugation (z. B. 15000 rpm), Lösungsmittelbehandlung, oder Ausfällen (z.B. mit Ammoniumsulfat), Filtration, Dialyse, Chromatographie und Kombinationen hiervon der Blätter oder Samen der Pflanzen. Dadurch werden die geeigneten Fraktionen mit den genannten Molekülmassen erhalten (ermittelbar über Gelelektrophorese), siehe auch US-A-5,776,470, EP- A 0 620 979 und darin genannte Literatur.

Beispielhaft kann hier folgende Methode zur Anwendung kommen:
100 - 200 g Hirse- Hafer- oder Weizen-Samenkörner werden in destilliertem Wasser unter Zusatz einer Pufferlösung (wie 0,2 M Ammoniumacetat, pH 5,4, Ammoniumphosphat) aufgenommen und mehrere Stunden bei Raumtemperatur homogenisiert. Anschließend wird die Mischung kalt Kurzzeit- zentrifugiert (z.B. bei 4 C, 12000 rpm, 20 Minuten). Der Überstand (Rohextrakt) wird sodann mit Ammoniumsulfat fraktioniert, zentrifugiert und der Niederschlag gesammelt, im Puffer aufgenommen (z. B. Acetat, Phosphat) und chromatographiert (z. B. an Sephadey C50, Gradientenelution mit NaCl, 200-400 mM im selben Puffer wie im Ausgangspuffer). Eine SDS-PAGE (Sodiumdodecylsulfat-polyacrylamide Gel Elektrophorese) - Analyse zeigt die geeigneten Fraktionen im Elutionsdiagramm an. Gewählt werden insbesondere solche mit einer mittleren Molekülmasse von 1 bis 10 kDa, vor allem von 1 bis 8 kDa und einem pH-Wert von insbesondere 4,5 bis 6,5 (20° C, 10%ige wässrige Lösung). Ein besonders geeignetes Pflanzenprotein ist Haferprotein mit einer mittleren Molekülmasse von 1 bis 1,3 kDa.

Milchproteine können erhalten werden auf bekannte Weise durch Ultrafiltration und Sprühtrocknung frischer Milch, wobei ein Trockenprodukt aus 78 bis 90 Gew.% Protein, Reste Lactose, Wasser, Asche erhalten wird. Hieraus können durch Chromatographie, insbesondere Anionenaustauschchromatographie, die geeigneten Proteine fraktioniert werden. Die geeigneten Substanzen weisen einen pH-Wert wie oben erläutert auf. Ein besonderes geeignetes Milchprotein ist solches mit einer mittleren Molekülmasse von ca. 1,8 bis 2,0 kDa.

Die so erhaltenen Proteinfraktionen werden überprüft auf ihre Lipidtransfereigenschaft. Dazu werden 2 Arten von Membranen verwendet: eine Donormemran und eine Akzeptormembran (Resonanz - Energie - Transfer Test (RET- Test), vgl. Nichols, J.W., Pagano R.E., J. Biol. Chem. 258 (1983), 5368-5371). Demnach können beispielsweise zwei Liposomenspezies unterschiedlicher Lipidzusammensetzung eingesetzt werden, wobei eine als Sonde verwendet wird und das Resonanz - EnergieTransfer- System mit zwei in Resonanz stehenden Fluorophoren, wie z. B. Nitrobenzoxadiazol und Rhodamin B enthält. Bei räumlicher Nähe der beiden Fluorophore in den Sondenliposomen veschwindet die Eigenfluoreszenz des angeregten Fluorophors, da die Energie vom zweiten Fluorophor unmittelbar absorbiert wird. Wird nun durch Protein - katalytischen Lipidtransfer der Abstand zwischen den beiden Fluorophoren verändert, so tritt die Eigenfluoreszenz des angeregten Fluorophors wieder auf. Sie ist ein direktes Maß für den Lipidtransfer. Die durch dieses Screnning aus den hergestellten Proteinfraktionen bzw. Hydrolysate hiervon erhaltenen Transferproteine können dann erfindungsgemäß eingesetzt werden.

Insbesondere sind die erfindungsgemäß eingesetzten Proteine zumindest wasserdispergierbar, vor allem aber wasserlöslich.

### 3. Trägerstoffe

Die dritte Komponente der erfindungsgemäßen Zubereitung umfasst Trägerstoffe, ausgewählt aus: Wasser; einem oder mehreren Zuckern/Zuckeraustauschstoffen; oder Mischungen hiervon. Die Zucker/Zuckeraustauschstoffe können gewählt werden aus Glucose, Maltose, Isomaltose, Fructose, Xylose, Lactose, Lactulose, Galactose, Inulin, Dextrose, Maltitol, Sorbitol, Xylitol, Mannitol, Erythritol, Zuckeraustauschstoffen (Isomalt, Maltitsirup), oder Mischungen hiervon, d.h. Mischungen aus verschiedenen Zuckern/ Zuckeraustauschstoffen, oder auch einem oder mehreren Zuckern und Wasser, insbesondere Sirupe mit einer Zuckerkonzentration von 30 bis 80 %, vorzugsweise 70% wie Glucosesirup, Saccharosesirup, 70%.

Die Trägerstoffe sind in Mengen von vor allem 68 bis 98 Gew.%, insbesondere 72 bis 95 Gew.%, vor allem 82 bis 95 Gew% vorhanden. Vor allem liegen als Trägerstoffe Gemische aus Wasser und einem oder mehreren Zuckem/Zuckeraustauschstoffen vor.

### 4. Zusatzstoffe

Als Zusatzstoffe, insbesondere pharmazeutische zulässige Zusatzstoffe für Medizin- und/oder Arzneimittel-Zubereitungen können ausgewählt werden:
- Lösungsmittel: Wasser, Glycerin, (Poly)Ethylenglykol; Polypropylenglykol; organische Lösungsvermittler wie oxylierte Fettsäuren, oxylierte Fettalkohole (Macrogole), Poloxamere (Polyethylenglykol-Polypropylen-Block-Copolymere wie z.B. solche unter dem Handelsnamen Lutrol® erhältliche Produkte mit geeigneter Molmasse);
- Verdicker: Arabisches Gummi; Cellulosederivate, z. B. Hydroxyethylcellulose, Hydroxypropylcellulose, Methylcellulose, Methylhydroxyethylcellulose; Dextrine wie Cyclodextrin, Maltodextrin, Dextrin Guargalactomannan, Dextran; Gelatine, Polydextrose, Chitosan, Chitosanhydrochlorid; Polyethylen-/propylenglycol; Gelatine; Kautschuk (Chicle, Apfelmilch) oder künstliche Kautschuk-Ersatzstoffe, Siliciumdioxid, Magnesiumstearat; Phospholipide (vor allem in Mengen von weniger als 1 Gew. %), z.B. aus Ei, Soja, Raps, Baumwollsamen, wie Phosphatidylcholin aus Soja oder Eigelb, Gemische mit Phosphatidylcholin in unterschiedlichen Verhältnissen wie NAT-Produkte, Phosphatidylethanolamin, Phosphatidylserin, Phosphatidylinositol aus Soja, Eigelb, Raps, Baumwollsamen, weiterhin Lecithine aus Soja, Eigelb, Raps, Baumwollsamen und diese Lecithin enthaltenden Lösungen, Dispersionen u.ä.; die Lipide (z. B. auch Glyceride, Öle, Kohlenwasserstoffe, Fettsäuren, Fett-säureester, Fettsäureether, Fettalkohole, Silikonöle), können insbesondere in Mengen von weniger als 1 Gew.% vorhanden sein, und sind bevorzugt nicht erforderlich; Zucker wie die vorstehend genannten, Saccharose; Salze wie Natriumchlorid, oder Mischungen aus vorgenannten Substanzen.
- Säuerungsmittel wie Zitronensäure, Weinsäure, Sorbinsäure; Kaliumcitrat, Puffer wie Natrium(hydrogen)-phosphat/carbonat, Salzsäure u.a.;
- Aroma- und Geschmacksstoffe wie Traubensaft-Konzentrat, Geranium Aroma NA, Johannisbeeraroma, Zitronenöl, Eukalyptusöl, Menthol; Orangenöl, Fenchelöl, Anisöl, Pfefferminzöl u.a.;
- Süßungsmittel, Zuckerersatzstoffe, insbesondere Sucralose; Honig; synthetische Süß-stoffe wie Aspartam; Saccharin-Na, oder Mischungen hiervon oder andere dem Fachmann zu diesem Zweck bekannte Süßungsmittel;
- Konservierungsmittel wie Kaliumsorbat, Methyl-Ethylparaben, Natriumbenzoat oder Gemische;
- Zusatzwirkstoffe wie insbesondere ein oder mehrere Wirkungsverstärkende vor allem auch terpenoide Inhaltsstoffe umfassende Substanzen (Zusatzwirkstoffe), ausgewählt aus Süßholzwurzel, Anis, Fenchel, Lindenblüten, Pimelwurzel, Sonnentaukraut, Efeublätter, Gelbwurz, Löwenzahnkraut, Efeu, Isländisch Moos oder Mischungen aus einem oder mehreren dieser Wirkungsverstärkenden Substanzen. Ggf. können noch andere wirksame oder pharmazeutisch wirksame Zusatzwirkstoffe wie beruhigende Stoffe wie Panthenol, Dexpanthenol, Vitamine eingesetzt werden.

Die Zusatzstoffe können in unterschiedlichen Mengen, insbesondere von 0 bis 30 Gew.%, vorzugsweise 0,1 bis 30, vorzugsweise 0,1 bis 25 Gew.%, insbesondere 0,1 bis 20 Gew.% , vor allem 0,1 bis 15 Gew.% vorliegen. Diese Mengenangaben sind bezogen auf die Gesamtzubereitung.

### Bevorzugte Ausführungsformen

Bevorzugt liegen 0,1 bis 20 Gew. % Zusatzstoffe in anmeldungsgemäßen Zubereitungen wie oben beschreiben vor.

Ganz besonders bevorzugt sind Zubereitungen, umfassend, oder auch bestehend aus, 0,1 bis 8 Gew. % Pflanzenwirkstoff(e), 0,1 bis 10 Gew.% Transferprotein, vor allem die vorstehend genannten, einen Träger aus, vorzugsweise 65 bis 95 oder 65 bis 74 Gew.%, Zucker/Zuckeraustauschstoffen, ausgewählt aus Glucose, Maltose, Isomaltose, Fructose, Dextrose, Xylose, Lactose, Lactulose, Galactose, Inulin, Maltitol, Sorbitol, Xylitol, Mannitol, Erythritol, Zuckerersatzstoffen, sowie Zusatzstoffen, ausgewählt aus Geschmacks- /Aromastoffen, Verdickern, insbesondere Cellulosederivaten, Poly(ethylen/Propylen)glykol, Konservierungsmitteln, Lösungsmitteln, Säuerungsmitteln, Puffersubstanzen, Zusatzwirkstoffen, wobei die Gesamtmenge an Wasser vorzugsweise weniger als 5 Gew. %, und die Gesamtmenge an Zucker maximal 95 Gew. % beträgt.

Die anmeldungsgemäße Zubereitung liegt bei derartigen Produkten vorzugsweise in fester bis gelförmiger Form vor, z. B. in Form von Pastillen wie ungefüllten oder gefüllten Pastillen, Gum-Pastillen, Medicated Gum, Kau-Dragees oder -Tabletten, insbesondere zum Lutschen. Es kann bei festen, insbesondere fest-gelförmigen Zubereitungsformen auch eine Aufteilung der Zusatz-/Trägerstoffe vorgenommen werden, so dass 2 Produktteile vorliegen: Dazu kann vorzugsweise in einem ersten Füllungsteil (I) die gesamte Mucobion-Kombination (Pflanzenwirkstoff, ggf. Zusatzwirkstoff + Lipidtransferprotein) vorliegen zusammen mit einem geringeren Teil der Trägerstoffe und Zusatzstoffen, insbesondere hier ausgewählt aus Zuckern, Wasser bzw. Lösungsmitteln wie Glycerin, Verdickern (wie Gelatine, Lecithin), während der zweite Hüllungsteil (II) die Hauptmenge der Träger- und Zusatzstoffe, ausgewählt aus Zucker/Zuckeraustauschstoffen, Wasser, bzw. Aroma-Geschmacksstoffen, Säuerungsmitteln, Puffersubstanzen, Konservierungsmitteln aufweist, wobei die Gesamtmenge an Wasser vor allem weniger als 5, vorzugsweise weniger als 3 Gew.% beträgt aufweist. Der Hüllungsteil mit der Hauptmenge an Zusatz-Trägerstoffen kann dabei vor allem fest sein, der erste Füllungsteil mit der geringeren Menge an Träger-/Zusatzstoffen kann fest bis flüssig, vor allem auch entsprechend der Wahl/dem Anteil an Verdicker, gelförmig, sein. Es kann so eine gefüllte Pastille erhalten werden. Bevorzugt ist der Hüllungsteil im Wesentlichen frei von insbesondere hochkalorischen Zuckern, und weist Zuckeraustausch/-ersatzstoffe auf.

Eine weitere bevorzugte Ausführungsform stellen Zubereitungen dar, umfassend, in s-besondere bestehend aus, 0,1 bis 15 Gew.%, vor allem 0.15 bis 8 Gew. % Phenolderivate enthaltender Pflanzen-Wirkstoff(en), 0,1 bis 15 Gew.%, vor allem 0,15 bis 10 Gew.% Transferprotein(en), vor allem die oben genannten bzw. die besonders bevorzugten wie oben beschrieben, und einem Träger aus 60 bis 90 Gew.% Wasser mit 40 bis 10 Gew.% oder 40 bis 0 Gew.%, vorzugsweise 40 bis 5 Gew.% Zucker/Zuckeraustauschstoffe, ausgewählt aus Glucose, Maltose, Isomaltose, Dextrose, Fructose, Xylose, Lactose, Lactulose, Galactose, Inulin, Maltitol, Sorbitol, Xylitol, Mannitol, Erythritol, sowie Zusatzstoffen, vorzugsweise ausgewählt aus Geschmacks-Aromastoffen, Verdickern, Konservierungsmitteln, Lösungsmitteln, Säuerungsmitteln, Puffersubstanzen, Zusatzwirkstoffen, wobei die Gesamtmenge an Wasser (in der Zubereitung) vorzugsweise 56 bis weniger als 90 Gew. % und die Menge an Zucker oder Zucker + Verdicker maximal 40 Gew. % beträgt. Dabei kann der Verdicker vor allem ausgewählt sein aus Poly(ethylen/-propylen)-glykol, Cellulosederivaten oder Gemischen hiervon.

Insbesondere können hier auch 85 bis weniger als 90 Gew. % Wasser und maximal 12 Gew.% Zucker oder Verdicker + Zucker, wie beschrieben vorhanden sein.

Die anmeldungsgemäße Zubereitung liegt in einer derartigen Ausführungsform vorzugsweise in flüssiger Form wie Elixier, Tropfen, Saft, Lösung (z.B. Mundwasser zum Gurgeln, Spray zum Sprühen zur jeweiligen lokalen Schleimhautapplikation im Mund- und Rachenraum) vor.

Bei geringerem Anteil an Wasser von weniger als 56% und entsprechendem Anteil an Zucker/Zuckeraustauschstoffen, Verdickern können Konzentrate oder dickflüssige Säfte erhalten werden. So kann aus einer Mischung aus 10-20 % Wasser + 40 bis 60% Zucker/-Zuckeraustauschstoffen, wie Glucosesirup ein für einen Hustensaft geeigneter Träger bereitgestellt werden, welcher in der Gesamtzubereitung in einer Menge von 50 bis 80 Gew. % vorhanden ist. Die Gesamtzubereitung umfasst, bzw. besteht insbesondere dann weiterhin (aus) geeignete Mengen an Wirkstoffen, Transferproteinen und Zusatzstoffen, wie insbesondere 0,1 bis 15 Gew.%, vor allem 0.15 bis 10 Gew, % Pflanzen-Wirkstoff-(en), 0,1 bis 8 Gew.%, vor allem 0,1 bis 8 Gew.% Transferprotein(en), vor allem die oben genannten bzw. die besonders bevorzugten wie oben beschrieben, sowie Zusatzstoffe, vorzugsweise ausgewählt aus Geschmacks-/Aromastoffen, Verdickern, Konservierungsmitteln, Lösungsmitteln, Süßungsmitteln, insbesondere Honig, Säuerungsmitteln, Puffersubstanzen, Zusatzwirkstoffen. Besonders bevorzugt sind Pastillen, insbesondere gefüllte Pastillen (Lutschbonbons), Saft variabler Konsistenz, Tropfen, und Lösungen.

Eine weitere vorteilhafte Ausgestaltung der vorliegenden Erfindung stellen Zubereitungen dar, worin der Phenol- Derivate-haltige Pflanzenstoff Pelargonium sidoides, bzw. Extrakte, Pulver hieraus und in einer Menge von 0,1 bis 10 Gew. % vorhanden ist, der oder die Trägerstoffe in Mengen von 62 bis 95 Gew.%, sowie 0,1 bis 27 Gew. % Zusatzstoffe vorhanden sind und das oder die Lipidtransferproteine in einer Menge von 0,1 bis 10 Gew. % vorhanden und ausgewählt sind aus Haferprotein, Weizenprotein, Gersteprotein oder Mischungen hiervon mit jeweils einer mittleren Molekülmasse von 1 bis 9 kDa, Milchprotein mit einer mittleren Molekülmasse von 1,5 bis 5 kDa, oder Kombinationen aus Hafer- und Milchprotein, Weizenprotein und Milchprotein, Gerste- und Milchprotein oder Hafer- und Gerste- und Milchprotein mit Molmassen wie beschrieben.

Ganz besonders bevorzugt ist in diesen anmeldungsgemäßen Kombinationen Pelargonium sidoides und/oder reniforme Wurzelextrakt (Trockenextrakt) mit einem Droge/Extrakt-Verhältnis von 3:1 bis 30:1, wie z.B. 7-25:1, vor allem mit Wasser/Ethanol als Extraktionsmittel (Wasser mit 10 bis 50% Ethanol, vor allem 10 bis 15% Ethanol).

Geeignet ist hier auch vor allem ein Trockenextrakt-Pulver hieraus, insbesondere mit 10 bis 70% nativem Extrakt, bevorzugt 10 Gew.%, und Maltodextrin als Zusatz.

Als Haupt-Trägerstoff (62-95%) ist hier insbesondere Wasser oder Zucker/Zuckeraustauschstoffe vorhanden.

### Anwendung

Die anmeldungsgemäßen Zubereitungen werden vorzugsweise eingesetzt zur mucosal/ lokalen (z. B. therapeutischen) Anwendung bei Menschen bei Irritationen und Reizungen der betreffenden Schleimhäute, welche aus Trockenheit, Entzündung, erkältungsbedingter, allergiebedingter oder durch Umweltpartikel bedingter Reizung oder entzündliche Zustände dadurch von Mund-Hals-Rachen-und/oder Bronchialschleimhäuten resultieren.

Insbesondere werden hierbei orale Anwendungsformen, wie pharmazeutische Anwendungsformen (zu denen Medizinprodukte, Arzneimittel gehören) eingesetzt. Dazu gehören: lutschbare Pastillen wie vor allem gefüllte Pastillen (Bonbons), Lutschtabletten, Kaudragees, Medicated Gums, oder ggf. versprühbare Lösungen wie Gurgel-Lösung, Mund-Was-ser, Mundspüllösung, Gele, Elixiere, Säfte, Tropfenapplikationen, Sirup. Dazu können vor allem die oben beschriebenen besonders bevorzugten Ausführungsformen (lutschbare Pastille, Saft, Tropfen) gewählt werden (je nach Wasseranteil fest oder flüssig). Die Zubereitungen können demnach pharmazeutische Zubereitungen darstellen, welche vor allem Medizinprodukte (therapeutisch, nicht therapeutisch) oder auch Arzneimittel, ggf. hier auch unter Zusatz von Zusatzwirkstoffen, umfassen, welche ebenfalls dem Begriff "pharmazeutische Zubereitung" zugeordnet werden. Bevorzugt liegen die Zubereitungen in Form von Medizinprodukten vor.

### Herstellung

Die anmeldungsgemäßen Zubereitungen werden hergestellt, indem bei flüssigen Produkten (Lösungen, Elixiere, Sprays, Säfte, Tropfen) die einzelnen Bestandteile vorzugsweise bei Raumtemperatur in das vorgelegte Lösungsmittel wie Wasser, und ggf. organische Lösungsmittel wie Propylenglykol, ggf. Alkohol unter Rühren hinzufügt werden. Bevorzugt werden die Phenolderivate enthaltenden pflanzlichen Wirkstoffe als Fluid eingesetzt. Im Falle von Gelen oder weniger dünnflüssigen Produkten wird der flüssigen Zubereitung ein geeigneter Verdicker hinzugesetzt.

Erhaltene Lösungen oder Gele können dann entsprechend der gewünschten Verabreichungsform in geeignete Applikatoren gefüllt werden und als Saft, Tropfen, Gurgel-lösung, eingesetzt werden.

So kann beispielsweise ein Saft erhalten werden aus Wasser, Zuckersirup, Honig, Aromen, Wirkstoffen, Transferproteinen, Zusätzen.

Bei festen Produkten werden die festen Grundlagen zusammen mit eventuell vorliegenden flüssigen Bestandteilen in geeigneten Mischvorrichtungen zusammengeben und dann je nach Vorgabe verpreßt, getrocknet (Tabletten, Dragees) . Pastillen können hergestellt wer-den, indem der Träger (z. B. flüssiges Isomalt, Maltesirpup) mit der Zubereitungsgrundmischung vermengt wird und anschließend in dafür bekannten Vorrichtungen als Strang aus-getragen und in geeignet große Teile geschnitten wird. Bei gefüllten Pastillen wird ein Füllungsteil mit der oben beschriebenen Grundmischung (z. B. enthaltend flüssiges Iso-malt) vermischt und sodann in einer geeigneten Vorrichtung (Doppelrohrführung) der Hüllungsteil (z.B. mit Maltesirup) eingefüllt. Beide Produktstränge werden dann gleichzeitig ausgetragen und portioniert.

Kautabletten können hergestellt werden, indem man der Basis (z.B. 75 bis 88 Gew.% Dextrose) die Zubereitungsmischung (Phenolderivate haltiger Wirkstoff, Transferprotein, vorzugsweise in pulvriger Form), Aromen, Hilfsmittel (wie Siliciumdioxid, Magnesiumstearat, Simethicon PhEur) in üblichen Apparaturen hinzufügt.

Medicated Gums werden mit Kautschuk, Chicle oder künstlichen Kautschukersatzstoffen aus den wässrigen Grundbestandteilen bereitet.

### Beispiele

In den nachfolgenden Beispielen sind anmeldungsgemäße Kombinationen (Beispiele 1-2) sowie feste Zubereitungen (Beispiel 3) und flüssige Zubereitungen (Beispiele 4-7) hiermit repräsentativ aufgezeigt. Die Beispiele 8, 9 belegen den Einfluss der erfindungsgemäßen Kombination/Zubereitung auf die Oberflächenspannung wässriger Systeme.

### Beispiel 1:

Es wurden 1 g gelbliches Pulver aus Pelargonium-Wurzelextrakt (festes Pulver, getrockneter Ethanol (10%)/Wasser-Extrakt, 30% nativ, Maltodextrin) + 1g Milchprotein, festes weißliches Pulver (mittlere Molekülmasse 2 kDa zusammengemischt. Man erhält ein schwach gelbliches trockenes Pulver.

### Beispiel 2: Flüssige Kombination:

In 100 ml Wasser wurden 1 g Milchprotein, mittlere Molekülmasse 1,8 kDa, 80%), und Pelargonium-Wurzel-Extrakt (Wasser +15 % Ethanol-Fluid-Extrakt (Droge-Extrakt Verhältnis von 1: 8), gemischt derart, dass das Verhältnis von Protein zu Pflanzenextrakt 3:1 ist. Man erhält eine leicht trübe bräunliche Flüssigkeit.

Derartige Kombinationen gemäß den Beispielen 1 bis 2 können direkt verwendet werden zur Herstellung anmeldungsgemäßer Zubereitungen. Alternativ können die Zubereitungen hergestellt werden durch jeweils separates Zumischen der einzelnen Inhaltsstoffe.

Die Zubereitungen gemäß den nachfolgenden Beispielen 3 bis 7 wurden durch jeweils separates Zumischen bei Raumtemperatur hergestellt.

### Beispiel 3: Feste Darreichungsform, Gefüllte Pastille

| Inhaltsstoffe | Rezeptur (in %) |
|---|---|
| Im Hüllungsteil I enthalten: | |
| Isomalt | 77 |
| Aromastoffe | 2 |
| Zitronensäure | 1,5 |

| Im Füllungsteil I enthalten | |
|---|---|
| Milchprotein (mittl. MW: 1,8 kDa) | 0,1 |
| Pelargoniumwurzelextrakt, trocken (Extr. Radix Pelargonii sidoidis, DEV 7-25:1, Wasser/15% ETOH, 10% nativ, Maltodextrin) | 0,3 |
| Aroma | 0,5 |
| Gelatine 100 Bloom | 0,5 |
| Maltisirup | 15 |
| Lecithin | 0,15 |
| Haferprotein, mittl. MW 1 kDa, Pulver | 0,1 |
| Glycerin | 1,3 |
| Restfeuchte | 1,55 |
| Gesamtmenge | 100% |

### Beispiel 4 Flüssige Darreichungsform: Tropfen

| Inhaltsstoffe | Rezeptur |
|---|---|
| Aqua purificata | 77,5 |
| Lutrol F 127 Ph. Eur. (Poloxamer) | 2,5 |
| Kaliumsorbat Ph.Eur. | 0,1 |
| Natriumbenzoat Ph.Eur. | 0,05 |
| Kaliumdihydrogenphosphat Ph.Eur. | 0,01 |
| Salzsäure 10 % Ph.Eur. | 0,34 |
| Gummi arabicum Ph.Eur. | 0,20 |
| Senegawurzel Trockenextrakt | 1,3 |
| Blanose 7H4F Ph. Eur. Carboxymethylcellulose, Na | 0,25 |
| Milchprotein (80% Gesamtprotein) MW 1,8 kDa | 0,3 |
| Haferprotein, MW 1,2 kDa | 0,8 |
| Soluthin MD (22% Phosphatidylcholine mit Maltodextrin) Pharmaz. Qualit. | 0,55 |
| Glycerin 85 % Ph.Eur. | 14,00 |
| Aroma | 1,00 |
| Pelargonium sid.-Trockenextrakt10%, Wasser/EtOH,15%) | 1,1 |
| Gesamtmenge | 100 % |

### Beispiel 5 Flüssige Darreichungsform: Tropfen

| Inhaltsstoffe | Rezeptur |
|---|---|
| Aqua purificata | 79,258 |
| Lutrol F 127 Ph. Eur. (Poloxamer) | 1,5 |
| Kaliumsorbat Ph.Eur. | 0,134 |
| Kaliumdihydrogenphosphat | 0,003 |
| Natriumbenzoat Ph.Eur. | 0,075 |
| Salzsäure, 10% | 0,54 |
| Gummi arabicum | 0,23 |
| Blanose 7H4F Ph. Eur. Carboxymethylcellulose, Na | 0,25 |
| Milchprotein (80% Gesamtprotein), fest MW 1,8 kDa | 0,23 |
| Haferprotein, fest (13 % Gesamtprotein) MW 1 kDa | 0,23 |
| Phytosolve (20% Triglyceride) | 0,55 |
| Glycerin 85 % Ph.Eur. | 15,00 |
| Aroma | 1,00 |
| Pelargonium-Trockenextrakt (wie Beispiel 4) | 1,00 |
| Gesamtmenge | 100 % |

### Beispiel 6 Flüssige Darreichungsform: Saft

| Rohstoffe | Rezeptur [%] |
|---|---|
| Aqua purificata | 18,00 |
| Kaliumsorbat Ph. Eur. | 0,11 |
| Natriumbenzoat Ph. Eur. | 0,06 |
| Pelargonium Trockenextrakt (analog Beispiel 7, aber 35% nativ) | 0,42 |
| Hyd roxyeth yl cell ulose HEC 6000 Ph. Eur | 0,50 |
| Milchprotein (80% Gesamtprotein), MW 1,8kDa | 0,24 |
| Haferprotein (50 % Gesamtprotein ), MW 1 kDa | 0,21 |
| Soluthin MD (22 % Phosphadidylcholine mit Maltodextrin) Pharmaz. Qualit. | 0,56 |
| Kaliumdihydrogenphosphat Ph.Eur. | 0,01 |
| Salzsäure 10 % Ph. Eur. | 0,52 |
| Gummi arabicum Ph. Eur. | 0,07 |
| Glucosesirup 70 % Ph. Eur. | 53,00 |
| Zuckersirup 70% | 10,00 |
| Glycerin 85 % Ph. Eur. | 4,00 |
| Aroma (Anisöl) | 1,2 |
| Aroma (Orangenöl) | 1,1 |
| Honig hell (Speisehonig, DAB) | 10,00 |
| Gesamtmenge | 100 g/% |

### Beispiel 7 Flüssige Darreichungsform: Saft

| Rohstoffe | Rezeptur [%] |
|---|---|
| Aqua purificata | 71,38 |
| Kaliumsorbat Ph. Eur. | 0,134 |
| Natriumbenzoat Ph. Eur. | 0,075 |
| Pelargonium Trockenextrakt (analog Beispiel 4) | 0,30 |
| Hyd roxyeth yl cell ulose HEC 6000 Ph. Eur | 0,50 |
| Milchprotein (80% Gesamtprotein, MW 1,8kDa) | 0,23 |
| Haferprotein (13 % Gesamtprotein, MW 1 kDa) | 0,23 |
| Phytosolv (Lecithin, 20% Triglyceride) | 0,55 |
| Kaliumdihydrogenphosphat Ph.Eur. | 0,003 |
| Salzsäure 10 % Ph. Eur. | 0,54 |
| Gummi arabicum Ph. Eur. | 0,06 |
| Glucosesirup 70 % Ph. Eur. | 10,00 |
| Zuckersirup 70% | 10,00 |
| Glycerin 85 % Ph. Eur. | 5,00 |
| Aroma | 1,00 |
| Gesamtmenge | 100 g/% |

| | |
|---|---|
| * Fluidextrakt, Wasser-Ethanol 50/50 , 70% | |

### Anwendungsbeispiel 8

Eine Testrezeptur TR (Mucobion-Rezeptur) gemäß Beispiel 5 , enthaltend 1 % nativen Extrakt aus Rad. Pelarg sid. (umfassend 5 % Gesamtphenolderivate), sowie 0,46 % Protein, je zur Hälfte aus Hafer und Milchprotein (Mucobion-Kombination aus Protein und Phenolderivate enthaltendem Pflanzenstoff) sowie Wasser und Zusatzstoffe wie in Beispiel 5 beschrieben wurde im Hinblick auf die Oberflächenspannung nach der Methode des hängenden Tropfens untersucht und nach der Laplace-Gleichung berechnet. Dabei wird die von der Oberflächenspannung abhängige charakteristische Form eines an einer nach unten gerichteten Kapillare hängenden Tropfens geometrisch vermessen.

Im vorliegenden Experiment, welche bei 20° C durchgeführt wurde, wurde das Meßgerät Easy Drop der Fa. Krüss verwendet. Die zu untersuchenden Testproben (Wasser, Speichel, Testrezeptur TR) wurden halbautomatisch in die Kanüle aufgezogen. An der Kapillare D=1,814 mm wurde durch Herausdrücken der Probenflüssigkeit ein Tropfen gebildet und mit Hilfe der Kamera optisch erfasst. Nach Erkennung einer korrekten Tropfenkontur durch die Software wurde die Oberflächenspannung durch diese automatisch berechnet.

Als Referenz-Standard diente einerseits die Oberflächenspannung von Wasser, da die Schleimschicht ganz überwiegend (96-99%) aus Wasser besteht. Als Vergleichs-Standard diente die Oberflächenspannung für Speichel, da Speichel ein wesentliche Komponente des Schleims der Mund- und Rachenhöhle ist.

Die Ergebnisse sind in nachfolgender Tabelle 1 angegeben:

**Tabelle 1**

| Substanz | Wasser (Ref.) | Speichel (Vgl.) | Testrezeptur (Erf.) |
|---|---|---|---|
| Oberflächenspannung (mN/m) | 71,1 | 57,7 | 38,7 |

### Anwendungsbeispiel 9

Nach der oben beschriebenen Methode wurde die Oberflächenspannung die Testrezeptur TR bei 20°C in unterschiedlichen Verdünnungen ermittelt und sowohl arithmetisch als auch als logarithmischer Wert berechnet.

Die Ergebnisse sind in den Abbildungen 1 (arithmetisch) und 2 (logarithmisch) dargestellt. Wie hieraus ersichtlich, wird erst ab einer 700- fachen Verdünnung der Testrezeptur die Oberflächenspannung von Speichel erreicht.

Diese Meßdaten belegen die Tatsache, dass durch die erfindungsgemäße Mucobion-Rezeptur die Oberflächenspannung der Schleimschicht erniedrigt wird, was unmittelbar zu einer verbesserten Schleimmotilität führt. Dadurch kann der Abtransport von Schadpartikeln erleichtert werden.

Gleichzeitig sind die phenolischen Verbindungen in der Mucobion-Rezeptur bereits durch die Proteine abgesättigt, so dass keine unerwünschten pharmakologischen immunologischen oder metabolischen Wechselwirkungen mit den Zellproteinen der Schleimhaut auftreten können.

Darüber hinaus zeigt auch das Verdünnungsexperiment, dass die Rezeptur über einen breiten Konzentrationsbereich einsetzbar ist, bzw. dass der überraschende Effekt auf die Mucobion-Kombination zurückzuführen ist.

## Patentansprüche

1. Lokal/oral einsetzbare Zubereitungen, umfassend eine Kombination aus einem oder mehreren Phenol- Derivate-haltigen Pflanzenstoffen, in Form von Extrakten oder Pulvern hieraus, gemäß der Formel 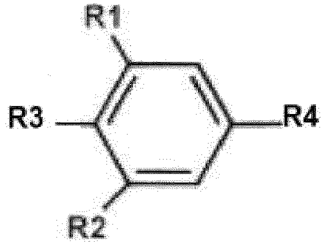 worin R₁, R₂, R₃, R₄, R₅ unabhängig voneinander bedeuten:
R₁: H, OH, OR₅, COR₅; C₁-C₈Alkyl, C₁-C₈substituiertes Alkyl;
R₂: OH; OR₅, H; C₁-C₈Alkyl, C₁-C₈substituiertes Alkyl;
R₃: OH; OR₅, C₁-C₈Alkyl, C₁-C₈substituiertes Alkyl; oder Zucker gemäß R_{4,5};
R₄: COOR₅; C₁-C₈-Alkyl, C₁-C₈-Alkenyl-COOR₅; C₁-C₈Alkyl, C₁-C₈substituiertes Alkyl;
R₅: H, C₁-C₈-Alkyl, COOC₁-C₈ alkyl-subst.Phenol, Zucker, ausgewählt aus Glucose,
Fructose, Fucose, Xylose, Galactose, Rhamnose, Xylose und Kombinationen aus 2 oder mehreren dieser Zucker; wobei die Zucker mit Mono-Di-Triterpenen assoziiert sein können,
ausgewählt aus Pelargonium sidoides und/oder reniforme oder Mischungen hiervon, oder aus Kombinationen Thymian + Spitzwegerich; Thymian + Spitzwegerich + Anis; Thymian + Primelwurzel; Pelargonium (sidoides, reniforme oder Mischungen) + Thymian und/oder Zistrose; Spitzwegerich + Efeublätter; Thymian + Fenchel; Spitzwegerich + Löwenzahn, Pelargonium (sidoides, reniforme oder Mischungen) + Zistrose und/oder Senegawurzel, Pelargonium + Granatapfel,
zusammen mit einem oder mehreren Lipidtransferproteinen, ausgewählt aus einem oder mehreren Pflanzenproteinen, ausgewählt aus Hafer, Hirse, Weizen, Gerste, Algen mit jeweils einer mittleren Molekülmasse von 1 bis 13 kDa, vorzugsweise von 1 bis 9 kDa, oder Milchprotein mit jeweils einer mittleren Molekülmasse von 1 bis 8 kDa, insbesondere 1,5 bis 7 kDa, oder Kombinationen hiervon (Pflanzen- und Milchprotein(en)),
sowie weiterhin Trägerstoffe, ausgewählt aus Wasser; einem oder mehreren Zuckern/Zuckeraustauschstoffen, oder Mischungen aus verschiedenen Zuckern/Zuckeraustauschstoffen oder auch aus einem oder mehreren Zuckern/Zuckeraustauschstoffen und Wasser, und ggf. Zusatzstoffe, ausgewählt aus Lösungsmitteln, Verdickern, Säuerungsmitteln, Puffersubstanzen, Aroma- und Geschmacksstoffen, Süßungsmitteln, Konservierungsmitteln, Zusatzwirkstoffen oder Mischungen aus einem oder mehreren dieser Zusatzstoffe.

2. Zubereitungen nach Anspruch 1, **dadurch gekennzeichnet, dass** die Zucker/Zuckeraustauschstoffe ausgewählt sind aus Glucose, Maltose, Isomaltose, Fructose, Xylose, Dextrose, Lactose, Lactulose, Galactose, Inulin, Maltitol, Sorbitol, Xylitol, Mannitol, Erythritol, oder Mischungen hiervon.

3. Zubereitungen nach Anspruch 1 oder 2, **dadurch gekennzeichnet, dass** der oder die Phenol- Derivate-haltigen Pflanzenstoffe in Form von Extrakten oder Pulvern in einer Menge von 0,1 bis 10 Gew. %, der oder die Trägerstoffe in Mengen von 72 bis 95 Gew.%, und das oder die Lipidtransferproteine in einer Menge von 0,1 bis 10 Gew. % sowie 0,1 bis 27 Gew. % Zusatzstoffe vorhanden sind.

4. Zubereitungen nach Anspruch 1, **dadurch gekennzeichnet, dass** der Phenol-Derivate-haltige Pflanzenstoff Pelargonium (sidoides, und/oder reniforme) in Form von Extrakten oder Pulvern und in einer Menge von 0,1 bis 10 Gew. % vorhanden ist, der oder die Trägerstoffe in Mengen von 62 bis 95 Gew.%, sowie 0,1 bis 27 Gew. % Zusatzstoffe vorhanden sind und das oder die Lipidtransferproteine in einer Menge von 0,1 bis 10 Gew. % vorhanden und ausgewählt sind aus Haferprotein, Weizenprotein, Gersteprotein oder Mischungen hiervon mit jeweils einer mittleren Molekülmasse von 1 bis 9 kDa, Milchprotein mit einer mittleren Molekülmasse von 1,5 bis 5 kDa, oder Kombinationen aus Hafer- und Milchprotein, Weizenprotein und Milchprotein, Gerste- und Milchprotein oder Hafer- und Gerste- und Milchprotein mit Molekülmassen wie beschrieben.

5. Zubereitungen nach Anspruch 4, **dadurch gekennzeichnet, dass** als Haupt-Trägerstoff (62-95%) Wasser oder Zucker/Zuckeraustauschstoffe vorhanden sind.

6. Zubereitungen nach einem der Ansprüche 1 bis 5, **dadurch gekennzeichnet, dass** sie 0,1 bis 10 Gew. % Phenol-Derivate-haltigen Pflanzenstoff(e) in Form von Extrakten oder Pulvern 0,1 bis 8 Gew.% Transferprotein(e), einen Träger aus 65 bis 95 Gew. % Zucker/, Zuckeraustauschstoffen, ausgewählt aus Glucose, Maltose, Dextrose, Isomaltose, Fructose, Xylose, Lactose, Lactulose, Galactose, Inulin, Maltitol, Sorbitol, Xylitol, Mannitol, Erythritol; und sowie 0,1 bis 20 Gew.% Zusatzstoffe aufweisen, wobei die Gesamtmenge an Wasser vorzugsweise weniger als 5 Gew. %, und die Gesamtmenge an Zucker maximal 95 Gew.% beträgt.

7. Zubereitungen nach Anspruch 6, **dadurch gekennzeichnet, dass** sie in fester bis gelförmiger Form in Form von (Lutsch)Pastillen, Dragees, Medicated Gums oder (Kau)Tabletten vorliegen.

8. Zubereitungen nach einem der Ansprüche 6 oder 7, **dadurch gekennzeichnet, dass** 2 Produktteile vorliegen, worin in einem ersten Füllungsteil (I) die gesamte Wirkstoffkombination aus Phenol-Derivate-haltigen Pflanzenstoff-(en) in Form von Extrakten oder Pulvern + ggf. Zusatzwirkstoff + Transferprotein(e) zusammen mit einem geringeren Teil der Träger-/Zusatzstoffe, insbesondere hier ausgewählt aus Wasser, Zucker/Zuckeraustauschstoffen bzw. Lösungsmittel wie Glycerin, Wasser, Verdickern (wie Gelatine, Lecithin), enthalten sind, während der zweite Hüllenteil (II) den Hauptteil der Träger-/Zusatzstoffe, ausgewählt aus Zucker/Zuckeraustauschstoffen, Lösungsmittel, Wasser, bzw. Aroma-Geschmacksstoffen, Säuerungsmitteln, Puffersubstanzen, aufweist.

9. Zubereitungen nach Anspruch 8, **dadurch gekennzeichnet, dass** Teil (II) vor allem fest und Teil (I) fest bis flüssig, vor allem gelförmig ist und die Zubereitung insbesondere in Form einer Pastille, Lutschpastille, vorliegt.

10. Zubereitungen nach einem der Ansprüche 1 bis 6, **dadurch gekennzeichnet, dass** sie 0,1 bis 10 Gew. % eines oder mehrerer Phenol-Derivate-haltigen Pflanzenstoff(en), in Form von Extrakten oder Pulvern 0,1 bis 8 Gew.% eines oder mehrerer Transferprotein(e), und einen Träger aus 60 bis 90 Gew. % Wasser und mit 40 bis 10 Gew.%, vorzugsweise 40 bis 5 Gew.% Zucker/ Zuckeraustauschstoffe, ausgewählt aus Glucose, Maltose, Dextrose, Isomaltose, Fructose, Xylose, Lactose, Lactulose, Galactose, Inulin, Maltitol, Sorbitol, Xylitol, Mannitol, Erythritol, Zuckerersatzstoffen, künstlichen Süßungsmitteln; sowie 0,1 bis 20 Gew.% Zusatzstoffe umfasst, wobei die Gesamtmenge an Wasser vorzugsweise 56 bis 90, vor allem 56 bis weniger als 90 Gew. % und die Menge an Zucker/Zuckeraustauschstoffe oder Zucker/ Zuckeraustauschstoffe + Verdicker maximal 40 Gew. % beträgt.

11. Zubereitungen nach Anspruch 10, **dadurch gekennzeichnet, dass** sie in Form einer Lösung (wie Gurgel-Mundspüllösung, Mundwasser, Elixier, Tinktur, Spray), einer Tropfen-Zubereitung, eines Geles, eines Konzentrates, eines Saftes zur flüssig lokal/oralen Applikation vorliegen.

12. Zubereitungen nach einem der Ansprüche 1 bis 11, **dadurch gekennzeichnet, dass** sie als Medizinprodukt vorliegt.

13. Zubereitungen gemäß einem der Ansprüche 1 bis 12 zur Anwendung bei Irritationen, Trockenheit, Entzündung, erkältungsbedingter, allergiebedingter oder durch Umweltpartikel bedingter Reizung oder entzündlichen Zuständen von Mund-Hals-Rachen-und/oder Nasal- oder Bronchialschleimhäuten.

## Claims

1. Locally/orally usable preparations comprising a combination of one or more phytochemicals containing phenol derivatives in the form of extracts or powders thereof according to the formula 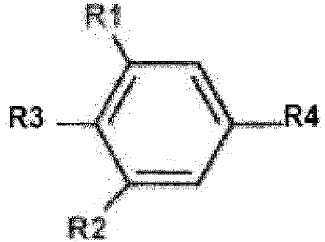 in which R₁, R₂, R₃, R₄, R₅ mutually independently mean:
R₁: H, OH, OR₅, COR₅; C₁-C₈ alkyl, C₁-C₈-substituted alkyl;
R₂: OH; OR₅, H; C₁-C₈ alkyl, C₁-C₈-substituted alkyl;
R₃: OH; OR₅, C₁-C₈ alkyl, C₁-C₈-substituted alkyl; or sugars according to R_{4,5};
R₄: COOR₅; C₁-C₈ alkyl, C₁-C₈-alkenyl-COOR₅; C₁-C₈ alkyl, C₁-C₈-substituted alkyl;
R₅: H, C₁-C₈ alkyl, COOC₁-C₈ alkyl-substituted phenols, sugars, selected from glucose, fructose, fucose, xylose, galactose, rhamnose and combinations of 2 or more of these sugars; wherein
the sugars may be associated with mono-, di- or triterpenes, selected from *Pelargonium sidoides* and/or *reniforme* or mixtures thereof, or of combinations thyme + ribwort; thyme + ribwort + aniseed; thyme + primula root; *Pelargonium* (*sidoides, reniforme* or mixtures) + thyme and/or rock rose; ribwort + ivy leaves; thyme + fennel; ribwort + dandelion, *Pelargonium* (*sidoides, reniforme* or mixtures) + rock rose and/or senega root, pelargonium + pomegranate,
together with one or more lipid transfer proteins selected from one or more plant proteins selected from oat, millet, wheat, barley, algae in each case having an average molecular mass of 1 to 13 kDa, preferably of 1 to 9 kDa, or milk protein in each case having an average molecular mass of 1 to 8 kDa, in particular of 1.5 to 7 kDa, or combinations thereof (plant and milk protein(s)),
as well as furthermore excipient substances, selected from water; one or more sugars/sugar substitutes, or mixtures of various sugars/sugar substitutes or also from one or more sugars/sugar substitutes and water, and optionally additives selected from solvents, thickeners, acidulants, buffer substances, aroma substances and flavours, sweeteners, preservatives, additional active ingredients or mixtures of one or more of these additives.

2. Preparations according to claim 1, **characterised in that** the sugars/sugar substitutes are selected from glucose, maltose, isomaltose, fructose, xylose, dextrose, lactose, lactulose, galactose, inulin, maltitol, sorbitol, xylitol, mannitol, erythritol, or mixtures thereof.

3. Preparations according to claim 1 or 2, **characterised in that** the phytochemical(s) containing phenol derivatives are present in the form of extracts or powders in a quantity of 0.1 to 10 wt.%, the excipient substance(s) is/are present in quantities of 72 to 95 wt.%, and the lipid transfer protein(s) in a quantity of 0.1 to 10 wt.%, as well as 0.1 to 27 wt.% additives.

4. Preparations according to claim 1, **characterised in that** the phytochemical containing phenol derivatives *Pelargonium* (*sidoides* and/or *reniforme*) is present in the form of extracts or powders and in a quantity of 0.1 to 10 wt.%, the excipient substance(s) is/are present in quantities of 62 to 95 wt.% as well as 0.1 to 27 wt.% additives, and the lipid transfer protein(s) is/are present in a quantity of 0.1 to 10 wt.% and are selected from oat protein, wheat protein, barley protein or mixtures thereof in each case having an average molecular mass of 1 to 9 kDa, and milk protein having an average molecular mass of 1.5 to 5 kDa, or combinations of oat and milk protein, wheat protein and milk protein, barley and milk protein or oat and barley and milk protein having molecular masses as described.

5. Preparations according to claim 4, **characterised in that** water or sugars/sugar substitutes are present as the main excipient substance (62-95%).

6. Preparations according to one of claims 1 to 5, **characterised in that** they comprise 0.1 to 10 wt.% phytochemical(s) containing phenol derivatives in the form of extracts or powders, 0.1 to 8 wt.% transfer protein(s), an excipient of 65 to 95 wt.% sugars/sugar substitutes, selected from glucose, maltose, dextrose, isomaltose, fructose, xylose, lactose, lactulose, galactose, inulin, maltitol, sorbitol, xylitol, mannitol or erythritol; as well as 0.1 to 20 wt.% additives, wherein the total quantity of water preferably amounts to less than 5 wt.% and the total quantity of sugars amounts to at most 95 wt.%.

7. Preparations according to claim 6, **characterised in that** they assume solid to gel form in the form of (suckable) pastilles, sugar-coated tablets, medicated gums or (chewable) tablets.

8. Preparations according to one of claims 6 or 7, **characterised in that** 2 product parts are present, a first filling part (I) containing the entire active ingredient combination of phytochemical(s) containing phenol derivatives in the form of extracts or powders + optionally additional active ingredient + transfer protein(s) together with a minor proportion of the excipient substances/additives, in particular in this case selected from water, sugars/sugar substitutes or solvents such as glycerol or water, thickeners (such as gelatin or lecithin), while the second shell part (II) comprises the major proportion of the excipient substances/additives, selected from sugars/sugar substitutes, solvents, water, or aroma substances/flavours, acidulants or buffer substances.

9. Preparations according to claim 8, **characterised in that** part (II) is primarily solid and part (I) solid to liquid, primarily in gel form, and the preparation in particular assumes the form of a pastille or suckable pastille.

10. Preparations according to one of claims 1 to 6, **characterised in that** they comprise 0.1 to 10 wt.% of one or more phytochemical(s) containing phenol derivatives in the form of extracts or powders, 0.1 to 8 wt.% of one or more transfer protein(s), and an excipient of 60 to 90 wt.% water and with 40 to 10 wt.%, preferably 40 to 5 wt.% sugars/sugar substitutes, selected from glucose, maltose, dextrose, isomaltose, fructose, xylose, lactose, lactulose, galactose, inulin, maltitol, sorbitol, xylitol, mannitol, erythritol, sugar substitutes and artificial sweeteners; as well as 0.1 to 20 wt.% additives, wherein the total quantity of water preferably amounts to 56 to 90, above all 56 to less than 90 wt.%, and the quantity of sugars/sugar substitutes or sugars/sugar substitutes + thickeners amounts to at most 40 wt.%.

11. Preparations according to claim 10, **characterised in that** they assume the form of a solution (such as gargle/mouthrinse solution, mouthwash, elixir, tincture or spray), a drop preparation, a gel, a concentrate or a succus for liquid local/oral administration.

12. Preparations according to one of claims 1 to 11, **characterised in that** they take the form of a medicine.

13. Preparations according to one of claims 1 to 12 for use in the case of irritation, dryness, inflammation, irritation caused by the common cold, allergies or environmental particles or inflammatory states of the oral/pharyngeal and/or nasal/bronchial mucosa.

## Revendications

1. Préparations utilisables localement/oralement, comprenant une combinaison d'une ou de plusieurs substances végétales contenant des dérivés du phénol, sous forme d'extraits ou de poudres de celle(s)-ci, selon la formule 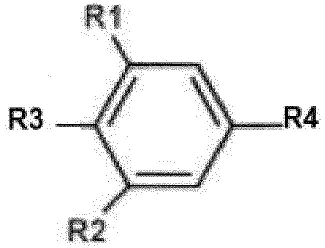 dans laquelle R₁, R₂, R₃, R₄, R₅ signifient, indépendamment les uns des autres :
R₁ : H, OH, OR₅, COR₅ ; C₁-C₈-alkyle, C₁-C₈-alkyle substitué ;
R₂ : OH ; OR₅, H ; C₁-C₈-alkyle, C₁-C₈-alkyle substitué ;
R₃ : OH ; OR₅, C₁-C₈-alkyle, C₁-C₈-alkyle substitué ; ou sucre selon R_{4,5} ;
R₄ : COOR₅ ; C₁-C₈-alkyle, C₁-C₈-alcényl-COOR₅ ; C₁-C₈-alkyle, C₁-C₈-alkyle substitué ;
R₅ : H, C₁-C₈-alkyle, phénol substitué par COOC₁-C₈-alkyle, sucre, choisi parmi le glucose, le fructose, le fucose, le xylose, le galactose, le rhamnose et des combinaisons de 2, ou plus, de ses sucres ; les sucres pouvant être associés à des monoterpènes, des diterpènes ou des triterpènes,
choisies parmi le Pelargonium sidoides et/ou reniforme ou des mélanges de ceux-ci ou parmi des combinaisons de thym + plantain lancéolé ; thym + plantain lancéolé + anis ; thym + racine de primevère ; Pelargonium (sidoides, reniforme ou mélanges) + thym et/ou ciste ; plantain lancéolé + feuilles de lierre ; thym + fenouil ; plantain lancéolé + pissenlit, Pelargonium (sidoides, reniforme ou mélanges) + ciste et/ou polygale de Virginie, Pelargonium + grenade, conjointement avec une ou plusieurs protéines de transfert lipidique, choisies parmi une ou plusieurs protéines végétales, choisies parmi l'avoine, le millet, le blé, l'orge, les algues, présentant à chaque fois une masse moléculaire moyenne de 1 à 13 kDa, de préférence de 1 à 9 kDa, ou une protéine de lait présentant à chaque fois une masse moléculaire moyenne de 1 à 8 kDa, en particulier de 1,5 à 7 kDa, ou des combinaisons de celles-ci (protéine(s) végétale(s) et de lait), ainsi que d'autres supports, choisis parmi l'eau ; un ou plusieurs sucres/substituts de sucre ou mélanges de différents sucres/substituts de sucre ou également de un ou plusieurs sucres/substituts de sucre et de l'eau, et le cas échéant des additifs, choisis parmi les solvants, les épaississants, les acidifiants, les substances tampon, les substances aromatisantes et gustatives, les édulcorants, les conservateurs, les substances actives supplémentaires ou des mélanges d'un ou de plusieurs de ces additifs.

2. Préparations selon la revendication 1, **caractérisées en ce que** les sucres/substituts de sucre sont choisis parmi le glucose, le maltose, l'isomaltose, le fructose, le xylose, le dextrose, le lactose, le lactulose, le galactose, l'inuline, le maltitol, le sorbitol, le xylitol, le mannitol, l'érythritol ou des mélanges de ceux-ci.

3. Préparations selon la revendication 1 ou 2, **caractérisées en ce que** la ou les substances végétales contenant des dérivés de phénol sont présents sous forme d'extraits ou de poudres en une quantité de 0,1 à 10% en poids, la ou les substances support en des quantités de 72 à 95% en poids et la ou les protéines de transfert lipidique en une quantité de 0,1 à 10% en poids et 0,1 à 27% en poids d'additifs.

4. Préparations selon la revendication 1, **caractérisées en ce que** la substance végétale contenant du phénol Pelargonium (sidoides, et/ou reniforme) est présente sous forme d'extraits ou de poudres et en une quantité de 0,1 à 10% en poids, la ou les substances support sont présentes en des quantités de 62 à 95% en poids et 0,1 à 27% en poids d'additifs sont présents et la ou les protéines de transfert lipidique sont présents en une quantité de 0,1 à 10% en poids et sont choisies parmi les protéines d'avoine, de blé, d'orge ou des mélanges de celles-ci, présentant à chaque fois une masse moléculaire moyenne de 1 à 9 kDa, une protéine de lait présentant une masse moléculaire moyenne de 1,5 à 5 kDa ou des combinaisons de protéines d'avoine et de lait, de protéines de blé et de lait, de protéines d'orge et de lait ou de protéines d'avoine et d'orge et de lait, présentant des masses moléculaires telles que décrites.

5. Préparations selon la revendication 4, **caractérisées en ce que** de l'eau ou des sucres/substituts de sucre sont présents en tant que substance support principale (62-95%).

6. Préparations selon l'une quelconque des revendications 1 à 5, **caractérisées en ce qu'**elle présentent 0,1 à 10% en poids de substance(s) végétale(s) contenant du phénol sous forme d'extraits ou de poudres, 0,1 à 8% en poids de protéine(s) de transfert, un support constitué par 65 à 95% en poids de sucres/substituts de sucre, choisis parmi le glucose, le maltose, le dextrose, l'isomaltose, le fructose, le xylose, le lactose, le lactulose, le galactose, l'inuline, le maltitol, le sorbitol, le xylitol, le mannitol, l'érythritol ; ainsi que 0,1 à 20% en poids d'additifs, la quantité totale d'eau étant de préférence inférieure à 5% en poids et la quantité totale de sucre(s) étant d'au maximum 95% en poids.

7. Préparations selon la revendication 6, **caractérisées en ce qu'**elles se trouvent sous forme de solide à gel, sous forme de pastilles (à sucer), de dragées, de gommes médicales ou de comprimés (à mâcher).

8. Préparations selon l'une quelconque des revendications 6 ou 7, **caractérisées en ce qu'**elles présentent 2 parties de produit, une première partie de remplissage (I) contenant la combinaison totale de substances actives constituée par une/des substance(s) végétale(s) contenant du phénol sous forme d'extraits ou de poudres + le cas échéant substance active supplémentaire + protéine(s) de transfert, conjointement avec une petite partie des substances support/additifs, en particulier choisi(e)s ici parmi l'eau, les sucres/substituts de sucre ou les solvants tels que le glycérol, l'eau, les épaississants (tels que la gélatine, la lécithine) alors que la deuxième partie (II) d'enveloppe présente la partie principale des substances support/additifs, choisi(e)s parmi les sucres/substituts de sucre, les solvants, l'eau ou les substances aromatisantes et gustatives, les acidifiants, les substances tampon.

9. Préparations selon la revendication 8, **caractérisées en ce que** la partie (II) est principalement solide et la partie (I) est solide à liquide, principalement sous forme de gel et la préparation se trouve en particulier sous forme d'une pastille, d'une pastille à sucer.

10. Préparations selon l'une quelconque des revendications 1 à 6, **caractérisées en ce qu'**elles comprennent 0,1 à 10% en poids d'une ou de plusieurs substance(s) végétale(s) contenant du phénol, sous forme d'extraits ou de poudres, 0,1 à 8% en poids d'une ou de plusieurs protéine(s) de transfert et un support constitué par 60 à 90% en poids d'eau et 40 à 10% en poids, de préférence 40 à 5% en poids de sucres/substituts de sucre, choisis parmi le glucose, le maltose, le dextrose, l'isomaltose, le fructose, le xylose, le lactose, le lactulose, le galactose, l'inuline, le maltitol, le sorbitol, le xylitol, le mannitol, l'érythritol, les substituts de sucre, les édulcorants artificiels ; ainsi que 0,1 à 20% en poids d'additifs, la quantité totale d'eau étant de préférence 56 à 90, principalement 56 à moins de 90% en poids et la quantité de sucres/substituts de sucre ou de sucres/substituts de sucre + épaississants étant d'au maximum 40% en poids.

11. Préparations selon la revendication 10, **caractérisées en ce qu'**elles se trouvent sous forme d'une solution (telles qu'une solution pour bain de bouche à gargariser, un bain de bouche, un élixir, une teinture, un spray), d'une préparation sous forme de gouttes, d'un gel, d'un concentrat, d'un jus pour l'application liquide locale/orale.

12. Préparations selon l'une quelconque des revendications 1 à 11, **caractérisées en ce qu'**elles se trouvent sous forme de produit médicinal.

13. Préparations selon l'une quelconque des revendications 1 à 12 pour une utilisation dans le cas d'irritations, de sécheresse, d'inflammation, d'irritation provoqués par le froid, une allergie ou des particules environnementales ou d'états inflammatoires des muqueuses de la bouche, de la gorge, du pharynx et/ou nasales ou bronchiales.
